# EUROPÄISCHE PATENTSCHRIFT

(11) **EP 2 229 406 B1**
(45) Veröffentlichungstag und Bekanntmachung des Hinweises auf die Patenterteilung: **22.04.2015**
(21) Anmeldenummer: 09700949.2
(22) Anmeldetag: 06.01.2009
(51) Int. Cl.: C07K 14/62

(54) **NEUE INSULINDERIVATE MIT EXTREM VERZÖGERTEM ZEIT- / WIRKUNGSPROFIL**
NOVEL INSULIN DERIVATIVES HAVING AN EXTREMELY DELAYED TIME-ACTION PROFILE
NOUVEAUX DÉRIVÉS D'INSULINE À PROFIL TEMPOREL/D'ACTION EXTRÊMEMENT RETARDÉ

(30) Priorität: 09.01.2008 DE 102008003566; 14.04.2008 US 44662 P; 24.05.2008 DE 102008025007
(43) Veröffentlichungstag der Anmeldung: 22.09.2010
(73) Patentinhaber: Sanofi-Aventis Deutschland GmbH, 65929 Frankfurt am Main (DE)
(72) Erfinder: HABERMANN, Paul, 65926 Frankfurt am Main (DE); SEIPKE, Gerhard, 65926 Frankfurt am Main (DE); KURRLE, Roland, 65926 Frankfurt am Main (DE); MÜLLER, Günter, 65926 Frankfurt am Main (DE); SOMMERFELD, Mark, 65926 Frankfurt am Main (DE); TENNAGELS, Norbert, 65926 Frankfurt am Main (DE); TSCHANK, Georg, 65926 Frankfurt am Main (DE); WERNER, Ulrich, 65926 Frankfurt am Main (DE)
(74) Vertreter: Heubeck, Christian
(86) Internationale Anmeldenummer: PCT/EP2009/000018
(87) Internationale Veröffentlichungsnummer: WO 2009/087082

(56) Entgegenhaltungen:
- WO-A-01/25278
- WO-A-90/11299
- WO-A-02/065985
- WO-A-02/079250
- WO-A-03/053339
- WO-A-2007/081824
- MARKUSSEN J ET AL: "SOLUBLE, PROLONGED-ACTING INSULIN DERIVATIVES. III. DEGREE OF PROTRACTION, CRYSTALLIZABILITY AND CHEMICAL STABILITY OF INSULINS SUBSTITUTED IN POSITIONS A21, B13, B23, B27 AND B30" PROTEIN ENGINEERING, OXFORD UNIVERSITY PRESS, SURREY, GB, Bd. 2, Nr. 2, 1. Juli 1988 (1988-07-01), Seiten 157-166, XP000006171 ISSN: 0269-2139

### SEQUENCE LISTING

<110> Sanofi-Aventis Deutschland GmbH
<120> Neue Insulinderivate mit extrem verzögertem Zeit- / Wirkungsprofil
<130> DE2008/002
<140> 102008003566.1-43
   <141> 2008-01-09
<160> 26
<170> PatentIn version 3.3
<210> 1
   <211> 23
   <212> PRT
   <213> Artificial
<220>
   <223> A-Kette
<220>
   <221> MISC_FEATURE
   <222> (1)..(1)
   <223> Xaa ist Lys, Arg oder einer Aminogruppe
<220>
   <221> MISC_FEATURE
   <222> (2)..(2)
   <223> Xaa ist Lys, Arg oder einer chemischen Bindung
<220>
   <221> MISC_FEATURE
   <222> (3) .. (3)
   <223> Xaa ist Arg oder Gly
<220>
   <221> MISC_FEATURE
   <222> (7)..(7)
   <223> Xaa ist Asp, Glu oder Gln
<220>
   <221> MISC_FEATURE
   <222> (17)..(17)
   <223> Xaa ist Asp, Glu oder Gln
<220>
   <221> MISC_FEATURE
   <222> (20) .. (20)
   <223> Xaa ist Asp, Glu oder Asn
<220>
   <221> MISC_FEATURE
   <222> (23) .. (23)
   <223> Xaa ist Ala, Ser, Thr oder Gly
<400> 1
   Xaa Xaa Xaa Ile Val Glu Xaa Cys Cys His Ser Ile Cys Ser Leu Tyr
   1               5                   10                  15
   Xaa Leu Glu Xaa Tyr Cys Xaa
               20
<210> 2
   <211> 34
   <212> PRT
   <213> Artificial
<220>
   <223> B-Kette
<220>
   <221> MISC_FEATURE
   <222> (1)..(1)
   <223> Xaa ist Asp, Glu oder eine Aminogruppe
<220>
   <221> MISC_FEATURE
   <222> (2) .. (2)
   <223> Xaa ist Asp, Glu oder eine chemische Bindung
<220>
   <221> MISC_FEATURE
   <222> (3) .. (3)
   <223> Xaa ist Asp, Glu, Phe oder eine chemische Bindung
<220>
   <221> MISC_FEATURE
   <222> (5) .. (5)
   <223> Xaa ist Asp, Glu oder Asn
<220>
   <221> MISC_FEATURE
   <222> (6) .. (6)
   <223> Xaa ist Asp, Glu oder Gln
<220>
   <221> MISC_FEATURE
   <222> (31) .. (31)
   <223> Xaa ist Arg, Lys oder eine Aminosäure ausgewählt aus einer Gruppe enthaltend die Aminosäuren Phe, Ala, Thr, Ser, Val, Leu, Glu oder Asp, oder einer chemischen Bindung
<220>
   <221> MISC_FEATURE
   <222> (32)..(32)
   <223> Xaa ist Thr oder eine chemische Bindung
<220>
   <221> MISC_FEATURE
   <222> (33) .. (33)
   <223> Xaa ist Arg, Lys oder eine chemische Bindung
<220>
   <221> MISC_FEATURE
   <222> (34) .. (34)
   <223> Xaa ist Arg-Amid oder Lys-Amid
<400> 2
   Xaa Xaa Xaa Val Xaa Xaa His Leu Cys Gly Ser His Leu Val Glu Ala
   1               5                   10                  15
   Leu Tyr Leu Val Cys Gly Glu Arg Gly Phe Phe Tyr Thr Pro Xaa Xaa
               20                  25                  30
   Xaa Xaa
<210> 3
   <211> 38
   <212> DNA
   <213> Artificial
<220>
   <223> pint3580_glya21rev
<400> 3
   caaaggtcga ctattagccg cagtagttct ccagctgg 38
<210> 4
   <211> 31
   <212> DNA
   <213> Artificial
<220>
   <223> arg_cjuncf
<400> 4
   gtccctgcag cgtcgcggca tcgtggagca g 31
<210> 5
   <211> 36
   <212> DNA
   <213> Artificial
<220>
   <223> arg_cjunc_rev
<400> 5
   ccacgatgcc gcgacgctgc agggacccct ccagcg 36
<210> 6
   <211> 32
   <212> DNA
   <213> Artificial
<220>
   <223> pint3580_Ha8f
<400> 6
   agcagtgctg ccacagcatc tgctccctct ac 32
<210> 7
   <211> 33
   <212> DNA
   <213> Artificial
<220>
   <223> pint3580_Ha8rev
<400> 7
   gagcagatgc tgtggcagca ctgctccacg atg 33
<210> 8
   <211> 31
   <212> DNA
   <213> Artificial
<220>
   <223> pint3581_Ea5f
<400> 8
   gcatcgtgga ggagtgctgc cacagcatct g 31
<210> 9
   <211> 32
   <212> DNA
   <213> Artificial
<220>
   <223> pint3581_Ea5rev
<400> 9
   ctgtggcagc actcctccac gatgccgcga cg 32
<210> 10
   <211> 47
   <212> DNA
   <213> Artificial
<220>
   <223> pint3580_Da18rev
<400> 10
   caaaggtcga ctattagccg cagtagtcct ccagctggta gagggag 47
<210> 11
   <211> 56
   <212> DNA
   <213> Artificial
<220>
   <223> pint3580_Ea15rev
<400> 11
   caaaggtcga ctattagccg cagtagttct ccagctcgta gagggagcag atgctg 56
<210> 12
   <211> 30
   <212> DNA
   <213> Artificial
<220>
   <223> pint3581_Db3f
<400> 12
   gcacgatttg tggaccagca cctgtgcggc 30
<210> 13
   <211> 34
   <212> DNA
   <213> Artificial
<220>
   <223> pint3581_Db3rev
<400> 13
   cacaggtgct ggtccacaaa tcgtgccgaa tttc 34
<210> 14
   <211> 30
   <212> DNA
   <213> Artificial
<220>
   <223> pint3581_Eb4f
<400> 14
   acgatttgtg aacgagcacc tgtgcggctc 30
<210> 15
   <211> 36
   <212> DNA
   <213> Artificial
<220>
   <223> pint3581_Eb4rev
<400> 15
   cgcacaggtg ctcgttcaca aatcgtgccg aatttc 36
<210> 16
   <211> 43
   <212> DNA
   <213> Artificial
<220>
   <223> pint3581_Eb0f1
<400> 16
   caacaggaaa ttcggcacga gagtttgtga accagcacct gtg 43
<210> 17
   <211> 44
   <212> DNA
   <213> Artificial
<220>
   <223> pint3581_Eb01f2
<400> 17
   tatcgaccat ggcaacaaca tcaacaggaa attcggcacg agag 44
<210> 18
   <211> 40
   <212> DNA
   <213> Artificial
<220>
   <223> pint3581_Eb0f1
<400> 18
   caacaggaaa ttcggcaaag tttgtgaacc agcacctgtg 40
<210> 19
   <211> 41
   <212> DNA
   <213> Artificial
<220>
   <223> pint3581_Eb01f2
<400> 19
   tatcgaccat ggcaacaaca tcaacaggaa attcggcaga g 41
<210> 20
   <211> 32
   <212> DNA
   <213> Artificial
<220>
   <223> 3702_arg_cjuncf
<400> 20
   tgcagaagcg cagaggcatc gtggagcagt gc 32
<210> 21
   <211> 32
   <212> DNA
   <213> Artificial
<220>
   <223> 3702_cjunc_rev
<400> 21
   tccacgatgc ctctgcgctt ctgcagggac cc 32
<210> 22
   <211> 32
   <212> DNA
   <213> Artificial
<220>
   <223> 3703_Δ Galf
<400> 22
   tgcagaagcg cagaatcgtg gagcagtgct gc 32
<210> 23
   <211> 32
   <212> DNA
   <213> Artificial
<220>
   <223> 3703_Δ Galrev
<400> 23
   tgctccacga ttctgcgctt ctgcagggac cc 32
<210> 24
   <211> 49
   <212> DNA
   <213> Artificial
<220>
   <223> pichia_G21_rev
<400> 24
   ttttttggcg ccgaattcac tattagccac agtagttttc cagctggta 49
<210> 25
   <211> 50
   <212> DNA
   <213> Artificial
<220>
   <223> pichia_H8_f
<400> 25
   gaacaatgtt gtcatagtat ctgttctttg taccagctgg aaaactactg 50
<210> 26
   <211> 35
   <212> DNA
   <213> Artificial
<220>
   <223> pichia-a1-12rev
<400> 26
   gaacagatac tatgacaaca ttgttcaacg atacc 35

## Beschreibung

Die Erfindung betrifft neue Insulinanaloga mit basalem Zeit- / Wirkungsprofil, ihre Herstellung und Verwendung.

Über die letzten Jahre hat die Zahl der Erkrankungen an Diabetes in einem geradezu epidemischen Ausmaß zugenommen. Aufgrund der Erkrankung kann es zu einer gravierenden Verkürzung der Lebenserwartung kommen. Menschen mit Diabetes müssen ihrem Körper oft Insulin von außen zuführen. Es ist sinnvoll, die Behandlung mit Insulin zu optimieren. Mittlerweile gibt es unterschiedliche Insuline mit spezifischen pharmakologischen Eigenschaften zur Behandlung. Praktischerweise werden die unterschiedlichen Insuline nach ihrer Wirkdauer unterschieden in kurzwirksame Insuline, schnellwirkende Insuline, langwirksame Insuline und Mischinsuline. Synonym verwendete Bezeichnungen für langwirksame Insuline sind Verzögerungsinsulin, Depotinsulin oder auch Basalinsulin. Die Wirkstoffe vieler dieser Insulinpräparate sind sogenannte Insulinanaloga, die vom humanen Insulin abgeleitet worden sind durch Substitution, Deletion und / oder Addition einer oder mehrerer Aminosäuren. Die Begriffe "Insulinanaloga" und "Insuline" werden hier synonym verwendet.

Das Konzept der intensivierten Insulintherapie versucht das Gesundheitsrisiko abzumindern, indem eine stabile Kontrolle des Blutzuckerspiegels durch frühe Gabe von Basalinsulinen angestrebt wird. Ein Beispiel für ein gängiges Basalinsulin ist das Medikament Lantus^{®} (Wirkstoff: Insulin Glargin = Gly (A21), Arg (B31), Arg (B32) Humaninsulin). Generell gilt es, bei der Entwicklung neuer, verbesserter Basalinsuline die Zahl hypoglykämischer Ereignisse zu minimieren. Ein ideales Basalinsulin wirkt dabei sicher in jedem Patienten mindestens 24 Stunden. Idealerweise setzt die Insulinwirkung verzögert und mit einem möglichst flachen Zeit-/ Wirkungsprofil ein, so dass die Gefahr einer kurzfristigen Unterzuckerung deutlich minimiert ist und die Applikation sogar ohne vorherige Einnahme von Nahrungsmitteln erfolgen kann. Eine gute Versorgung mit Basalinsulin ist dann gegeben, wenn die Insulinwirkung möglichst lange gleichbleibend anhält, d.h. der Körper mit einer konstanten Menge Insulin versorgt wird. Damit ist die Gefahr hypoglykämischer Ereignisse gering und eine patienten- und tagesspezifische Variabilität minimiert. Das pharmakokinetische Profil eines idealen Basalinsulins sollte also durch einen verzögerten Wirkeintritt und durch eine verzögerte, d.h. lang anhaltende und gleichmäßige Wirkung gekennzeichnet sein.

Jedoch zeigt - trotz der bereits erreichten therapeutischen Vorteile - keines der bisher beschriebenen Verzögerungsinsuline die pharmakokinetischen Eigenschaften eines idealen Basalinsulins. Wünschens-wert sind Insuline, die ein solch flaches und lang andauerndes Zeit- / Wirkungsprofil haben, dass die Gefahr hypoglykämischer Ereignisse und der tagesabhängigen Varianz im Patienten weiter minimiert ist und die Wirkdauer weiter verzögert ist, so dass unter Umständen nicht mehr täglich Insulin verabreicht werden muss. Dies würde eine vereinfachte Behandlung von Diabetikern ermöglichen, insbesondere von älteren und pflegebedürftigen Diabetikern, die sich Insulin nicht mehr selber injizieren können und wäre somit auch von großem volkswirtschaftlichem Nutzen. In der frühen Phase des Diabetes Typ 2 wären solche Basalinsuline zudem nützlich. Kliniker berichten, dass die bei vielen Menschen vorhandene Phobie vor Spritzen sie davor zurückschrecken lässt, rechtzeitig mit der Insulintherapie zu beginnen. Als Konsequenz ergibt sich eine schlechte Blutzuckereinstellung, die diabetische Spätfolgen nach sich zieht. Ein Basalinsulin, das die Anzahl der durch Spritzen erfolgten Insulingaben vermindert, könnte bewirken, dass Patienten leichter die Insulintherapie annehmen.

Kohn et al. (Peptides 28 (2007) 935-948) beschreiben, dass man die Optimierung der Pharmakodynamik von Insulin dadurch erreichen kann, dass man Insulinanaloga herstellt, deren isoelektrischer Punkt (pl) durch Addition von Lysin oder Arginin an das B- Kettenende oder den N-Terminus der A und B- Kette in Richtung des alkalischen Bereichs verschoben ist, verglichen mit dem isoelektrischen Punkt von Humaninsulin (pl = 5,6), so dass die Löslichkeit unter physiologischen Bedingungen verringert ist und sich ein verlängertes Zeit- / Wirkungsprofil ergibt. Verbindung 18 aus Kohn et al. (Arg (A0), Gly (A21), Arg (B31), Arg (B32) Humaninsulin (pl experimentell bestimmt = 7,3; pl berechnet = 7,58) wird dabei als beste Verbindung im Sinne des Konzeptes dargestellt. Das hauptsächliche Ziel bei der Gestaltung neuer Insulinanaloga sehen Kohn et al. daher in der Addition von positiv geladenen Aminosäuren zur Aminosäuresequenz von Humaninsulin zwecks Erhöhung des isoelektrischen Punktes von pl = 5,6 in den neutralen Bereich.

Es wurde nun aber überraschend gefunden, dass solche Insulinanaloga zu dem beschriebenen wünschenswerten basalen Zeit- / Wirkungsprofil führen, die durch die Merkmale charakterisiert sind, dass
- das B- Kettenende aus einem amidierten basischen Aminosäurerest wie Argininamid besteht, d.h. bei dem amidierten basischen Aminosäurerest am B-Kettenende liegt die Carboxylgruppe der endständigen Aminosäure in ihrer amidierten Form vor, und
- die Aminosäureposition A8 durch einen Histidinrest besetzt wird, und
- die Aminosäureposition A21 durch einen Glycinrest besetzt wird, und
- nicht mehr als ein Aminosäurerest der Gruppe enthaltend A5, A15, A18, B-1, B0, B1, B2, B3 und B4Asp oder Glu entspricht.

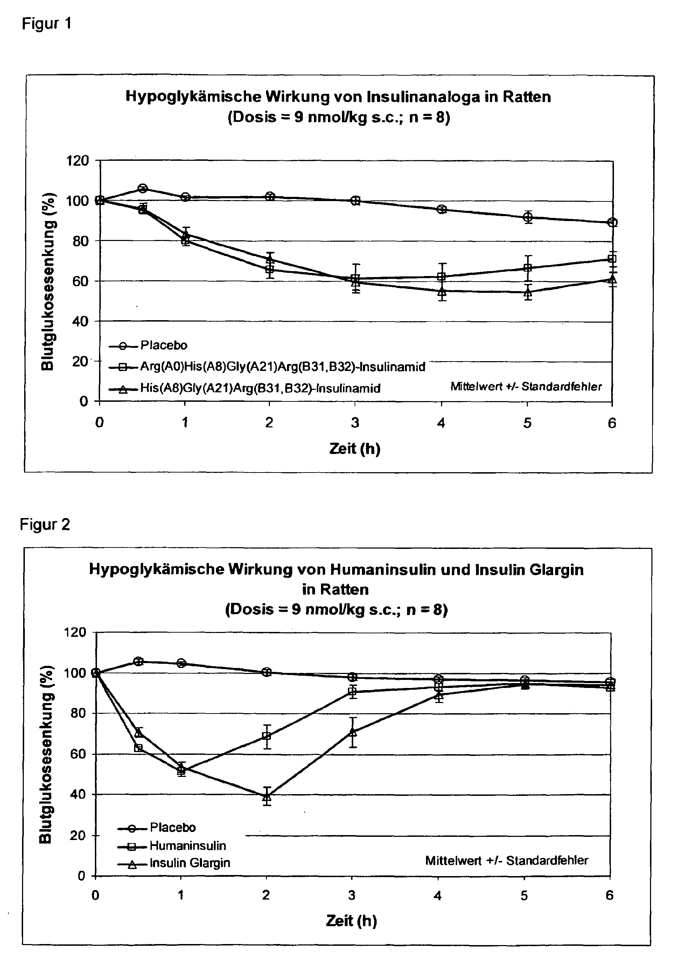

Überraschenderweise haben gerade die beschriebenen Insulinanaloga die gewünschten vorteilhaften dem Ideal nahe kommenden Zeit - / Wirkungsprofile, d.h. einen verzögerten flachem Wirkeintritt und eine längere Wirkdauer auf. Damit wird die Gefahr von hypoglykämischen Ereignissen deutlich minimiert. Die Verzögerung ist so deutlich, dass sich der Effekt überraschend sogar in Modellversuchen an Ratten nachweisen lässt. Im Gegensatz dazu ist die verzögerte Wirkung von Insulin Glargin, wie Figur 2 zeigt, in der Ratte nicht eindeutig zu beobachten. In Figur 1 ist die hyopglykämische Wirkung der erfindungsgemäßen Verbindung YKL202 und YKL203 dargestellt. Die im Rattenversuch erzielten Ergebnisse werden im Hundeversuch bestätigt. Auch hier wird eine deutlich verlängerte Wirkdauer gegenüber Insulin Glargin beobachtet. Damit sind neue Basalinsuline bereitgestellt worden, die deutlich weniger häufig appliziert werden müssen. Neben diesen beschriebenen pharmakokinetischen Vorteilen weisen die erfindungsgemäßen Analoga gegenüber Insulin Glargin bessere Eigenschaften in pharmakokinetischer Hinsicht auf. Zudem zeigen die beanspruchten Insuline auch in physikalisch - chemischer Hinsicht Vorteile.

Es wurde nun überraschenderweise gefunden, dass Insulinanaloga der Formel I 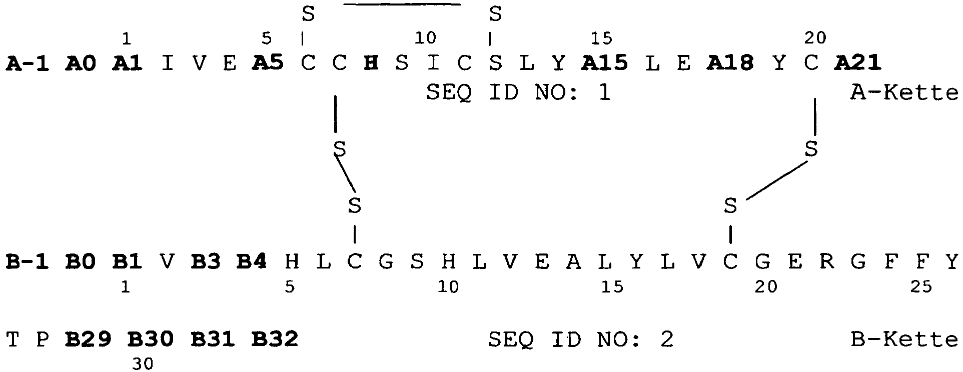 wobei
A-1 Lys, Arg oder einer Aminogruppe;
A0 Lys, Arg oder einer chemischen Bindung;
A1 Arg oder Gly;
A5 Asp, Glu oder Gln;
A15 Asp, Glu oder Gln;
A18 Asp, Glu oder Asn;
A21 Ala, Ser, Thr oder Gly;
B-1 Asp, Glu oder eine Aminogruppe;
B0 Asp, Glu oder eine chemische Bindung;
B1 Asp, Glu, Phe oder eine chemische Bindung;
B3 Asp, Glu oder Asn;
B4 Asp, Glu oder Gln;
B29 Arg, Lys oder einer Aminosäure ausgewählt aus einer Gruppe enthaltend die Aminosäuren Phe, Ala, Thr, Ser, Val, Leu, Glu oder Asp, oder einer chemischen Bindung;
B30 Thr oder einer chemischen Bindung;
B31 Arg, Lys oder einer chemischen Bindung;
B32 Arg-Amid oder Lys-Amid
entspricht, wobei nicht mehr als ein Aminosäurerest der Gruppe enthaltend A5, A15, A18, B-1, B0, B1, B2, B3 und B4 Asp oder Glu entspricht, das gewünschte pharmakologische Profil, d.h. einen verzögerten Wirkeintritt und durch eine länger anhaltende und gleichmäßige Wirkung, haben.

Die vorliegende Erfindung betrifft Insulinanaloga ausgewählt aus der Gruppe enthaltend
Arg (A0), His (A8), Gly (A21), Arg (B31), Arg (B32) - NH₂ - Humaninsulin,
Arg (A0), Glu (A15), His (A8), Gly (A21), Arg (B31), Arg (B32) - NH₂ - Humaninsulin,
His (A8), Gly (A21), Arg (B31), Arg (B32) - NH₂ - Humaninsulin.

Untenstehende Insulinanaloga werden beschrieben:
Arg (A-1), Arg (A0), Glu (A5), His (A8), Gly (A21), Arg (B30) - NH₂ Humaninsulin,
Arg (A-1), Arg (A0), Glu (A5), His (A8), Gly (A21), Lys (B30) - NH₂ Humaninsulin,
Arg (A-1), Arg (A0), Glu (A15), His (A8), Gly (A21), Arg (B30) - NH₂ Humaninsulin,
Arg (A-1), Arg (A0), Glu (A15), His (A8), Gly (A21), Lys (B30) - NH₂ Humaninsulin,
Arg (A-1), Arg (A0), Asp (A18), His (A8), Gly (A21), Arg (B30) - NH₂ Humaninsulin,
Arg (A-1), Arg (A0), Asp (A18), His (A8), Gly (A21), Arg (B30) - NH₂ Humaninsulin,
Arg (A-1), Arg (A0), His (A8), Gly (A21), Glu (B0), Arg (B30) - NH₂ Humaninsulin,
Arg (A-1), Arg (A0), His (A8), Gly (A21), Glu (B0), Lys (B30) - NH₂ Humaninsulin,
Arg (A-1), Arg (A0), His (A8), Gly (A21), Asp (B3), Arg (B30) - NH₂ Humaninsulin,
Arg (A-1), Arg (A0), His (A8), Gly (A21), Asp (B3), Lys (B30) - NH₂ Humaninsulin,
Arg (A-1), Arg (A0), His (A8), Gly (A21), Glu (B4), Arg (B30) - NH₂ Humaninsulin,
Arg (A-1), Arg (A0), His (A8), Gly (A21), Glu (B4), Lys (B30) - NH₂ Humaninsulin,
Arg (A0), His (A8), Gly (A21), Arg (B31), Lys (B32) - NH₂ - Humaninsulin,
Arg (A0), Glu (A5), His (A8), Gly (A21), Arg (B31), Arg (B32) - NH₂ - Humaninsulin,
Arg (A0), Glu (A5), His (A8), Gly (A21), Arg (B31), Lys (B32) - NH₂ - Humaninsulin,
Arg (A0), Asp (A18), His (A8), Gly (A21), Arg (B31), Arg (B32) - NH₂ - Humaninsulin,
Arg (A0), Asp (A18), His (A8), Gly (A21), Arg (B31), Lys (B32) - NH₂ - Humaninsulin,
Arg (A0), Glu (A15), His (A8), Gly (A21), Arg (B31), Lys (B32) - NH₂ - Humaninsulin,
Arg (A0), His (A8), Gly (A21), Asp (B3), Arg (B31), Arg (B32) - NH₂ - Humaninsulin,
Arg (A0), His (A8), Gly (A21), Asp (B3), Arg (B31), Lys (B32) - NH₂ - Humaninsulin,
Arg (A0), His (A8), Gly (A21), Glu (B4), Arg (B31), Arg (B32) - NH₂ - Humaninsulin,
Arg (A0), His (A8), Gly (A21), Glu (B4), Arg (B31), Lys (B32) - NH₂ - Humaninsulin,
Arg (A0), His (A8), Gly (A21), Glu (B0), Arg (B31), Arg (B32) - NH₂ - Humaninsulin,
Arg (A0), His (A8), Gly (A21), Glu (B0), Arg (B31), Lys (B32) - NH₂ - Humaninsulin,
Arg (A0), His (A8), Gly (A21), Arg (B30) - NH₂ - Humaninsulin,
Arg (A0), His (A8), Gly (A21), Lys (B30) - NH₂ - Humaninsulin,
Arg (A-1), Arg (A0), His (A8), Gly (A21), Arg (B30) - NH₂ - Humaninsulin,
Arg (A-1), Arg (A0), His (A8), Gly (A21), Lys (B30) - NH₂ - Humaninsulin,
Arg (A0), Arg (A1), His (A8), Gly (A21), Arg (B30) - NH₂ - Humaninsulin,
Arg (A0), Arg (A1), His (A8), Gly (A21), Lys (B30) - NH₂ - Humaninsulin.

Durch die Angabe des Begriffs "Humaninsulin" in den Bezeichnungen der genannten Insulinanaloga wird Bezug auf die Aminosäuresequenzen der A- und B-Kette von Humaninsulin genommen und alle Abweichungen (Additionen, Substitutionen, Deletionen) davon sind in einer gegebenen Bezeichnung eines Insulinanalogons angegeben.

Ein weiterer Gegenstand der Erfindung ist ein Verfahren zur Herstellung eines Insulinanalogons wie oben beschrieben, wobei ein Vorläufer des Insulinanalogs rekombinant hergestellt wird, der Vorläufer enzmatisch zu zwei -kettigem Insulin prozessiert wird und eine Kupplung mit Argininamid in Gegenwart eines Enzyms mit Trypsinaktivität durchgeführt wird, und das Insulinanalogon isoliert wird.

Ein weiterer Gegenstand der Erfindung ist die Verwendung eines Insulinanalogons wie oben beschrieben, zur Herstellung eines Medikaments zur Behandlung von Diabetes Mellitus., insbesondere von Diabetes MellitusTyp I oder Typ II.

Ein weiterer Gegenstand der Erfindung ist ein Arzneimittel enthaltend ein Insulinanalogon wie oben beschrieben und/oder physiologisch verträgliche Salze davon.

Ein weiterer Gegenstand der Erfindung ist ein Arzneimittel enthaltend ein Insulinanalog wie oben beschrieben, das therapeutisch zur Knorpelregeneration eingesetzt wird.

Ein weiterer Gegenstand der Erfindung ist ein Arzneimittel enthaltend ein Insulinanalog wie oben beschrieben, das therapeutisch zur Unterstützung der beta - Zellregeneration eingesetzt wird.

Ein weiterer Gegenstand der Erfindung ist eine Formulierung des Insulinanalogons wie oben beschrieben, wobei die Formulierung in wässriger Form enthaltend das gelöste Insulinanalogon vorliegt.

Ein weiterer Gegenstand der Erfindung ist eine Formulierung des Insulinanalogons wie oben beschrieben, wobei die Formulierung in Form von Pulver vorliegt, insbesondere in kristalliner und/oder amorpher Form.

Ein weiterer Gegenstand der Erfindung ist eine Formulierung des Insulinanalogons wie oben beschrieben, wobei die Formulierung in Form einer Suspension vorliegt. Ein weiterer Gegenstand der Erfindung ist eine Formulierung des Insulinanalogons wie oben beschrieben, wobei die Formulierung zusätzlich ein chemisches Chaperon enthält.

Eine DNA kodierend für einen Vorläufer eines Insulinanalogons wie oben beschrieben wird beschrieben,

Eine DNA kodierend für die A-Kette eines Insulinanalogons wie oben beschrieben wird beschrieben.

Eine DNA kodierend für die B-Kette eines Insulinanalogons wie oben beschrieben wird beschrieben.

Ein Vektor enthaltend eine DNA wie oben beschrieben wird beschrieben.

Ein Wirtsorganismus enthaltend eine DNA wie oben beschrieben oder einen Vektor wie oben beschrieben wird berichtet.

Ein Präproinsufinanalogon, dadurch gekennzeichnet, dass das C-Peptid an seinem N-Terminus den Aminosäurerest Arginin trägt und sein C-Terminus durch die Form Arg Arg, Arg Lys oder Lys Arg Arg gekennzeichnet ist, wird beschrieben.

Ein weiterer Gegenstand der Erfindung ist eine Formulierung wie oben beschrieben, bei der noch zusätzlich ein Glucagon-Like Peptide-1 (GLP1) oder ein Analogon oder Derivat davon, oder Exendin-3 bzw. -4 oder ein Analogon oder Derivat davon, vorzugsweise Exendin-4 enthalten ist.

Ein weiterer Gegenstand der Erfindung ist eine Formulierung wie oben beschrieben, bei dem ein Analogon von Exendin-4 ausgewählt wird aus einer Gruppe enthaltend
H-desPro³⁶-Exendin-4-Lys₆-NH₂,
H-des(Pro^{36,37})-Exendin-4-Lys₄-NH₂ und
H-des(Pro^{36,37})-Exendin-4-Lys₆-NH₂, oder ein pharmakologisch tolerierbares Salz davon.

Ein weiterer Gegenstand der Erfindung ist eine Formulierung wie oben beschrieben, bei dem ein Analogon von Exendin-4 ausgewählt wird aus einer Gruppe enthaltend
desPro³⁶ [Asp²⁸]Exendin-4 (1-39),
desPro³⁶ [IsoAsp²⁸]Exendin-4 (1-39),
desPro³⁶ [Met(O)¹⁴, Asp²⁸]Exendin-4 (1-39),
desPro³⁶ [Met(O)¹⁴, IsoAsp²⁸]Exendin-4 (1-39),
despro³⁶ [Trp(O₂)²⁵, Asp²⁸]Exendin-2 (1-39),
desPro³⁶ [Trp(O₂)²⁵, IsoAsp²⁸]Exendin-2 (1-39),
desPro³⁶ [Met(O)¹⁴Trp(O²)²⁵, Asp²⁸]Exendin-4 (1-39) und
desPro³⁶ [Met(O)¹⁴Trp(O₂)²⁵, IsoAsp²⁸]Exendin-4 (1-39),
oder ein pharmakologisch tolerierbares Salz davon.

Ein weiterer Gegenstand der Erfindung ist eine Formulierung wie im vorigen Absatz beschrieben, bei denen an die C-Termini der Analoga von Exendin-4 das Peptid - Lys₆-NH₂ angefügt ist.

Ein weiterer Gegenstand der Erfindung ist eine Formulierung wie oben beschrieben, bei dem ein Analogon von Exendin-4 ausgewählt wird aus einer Gruppe enthaltend
H-(Lys)₆- des Pro³⁶ [Asp²⁸]Exendin-4(1-39)-Lys₆-NH₂
des Asp²⁸Pro³⁶, Pro³⁷, Pro₃₈ Exendin-4(1-39) -NH₂,
H-(Lys)₆- des Pro³⁶, Pro³⁷, Pro³⁸ [Asp²⁸]Exendin-4(1-39) -NH₂,
H-Asn-(Glu)₅ des Pro³⁶, Pro³⁷, Pro³⁸ [Asp²⁸]Exendin-4(1-39) -NH₂,
des Pro³⁶, Pro³⁷, Pro³⁸ [Asp²⁸]Exendin-4(1-39)-(Lys)₆-NH₂,
H-(Lys)₆- des Pro³⁶, Pro³⁷, Pro³⁸ [Asp²⁸]Exendin-4(1-39)-(Lys)₆-NH₂,
H-Asn-(Glu)₅- des Pro³⁶, Pro³⁷, Pro³⁸ [Asp²⁸]Exendin-4(1-39)-(Lys)₆-NH₂,

H-(Lys)₆- des Pro³⁶ [Trp(O₂)²⁵, Asp²⁸]Exendin-4(1-39)-Lys₆-NH₂,
H- des Asp²⁸ Pro³⁶, Pro³⁷, Pro³⁸ [Trp(O₂)²⁵]Exendin-4(1-39) -NH₂,
H-(Lys)₆- des Pro³⁶, Pro³⁷, Pro³⁸ [Trp(O₂)²⁵, Asp²⁸]Exendin-4(1-39) -NH₂,
H-Asn-(Glu)₅- des Pro³⁶, Pro³⁷, Pro³⁸[Trp(O₂)²⁵, Asp²⁸]Exendin-4(1-39) -NH₂,
des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶, Pro³⁷, Pro³⁸ [Trp(O₂)²⁵, Asp²⁸]Exendin-4(1-39)-(Lys)₆-NH₂,
H-Asn-(Glu)₅- des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶ [Met(O)¹⁴, Asp²⁸]Exendin-4(1-39)-Lys₆-NH₂,
des Met(O)¹⁴ Asp²⁸ Pro³⁶, Pro³⁷, Pro³⁸ Exendin-4(1-39) -NH₂,
H-(Lys)₆- des Pro³⁶, Pro³⁷, Pro³⁸ [Met(O)¹⁴, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Asp²⁸]Exendin-4(1-39)-(Lys)₆-NH₂,
H-(Lys)₆- des Pro³⁶, Pro³⁷, Pro³⁸ [Met(O)¹⁴, Asp²⁸]Exendin-4(1-39)-Lys₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,

H-(Lys)₆- des Pro³⁶ [Met(O)¹⁴, Trp(O₂)²⁵, Asp²⁸]Exendin-4(1-39)-Lys₆-NH₂,
des Asp²⁸ Pro³⁶, Pro³⁷, Pro³⁸ [Met(O)¹⁴, Trp(O₂)²⁵]Exendin-4(1-39) -NH₂,
H-(Lys)₆- des Pro³⁶- Pro³⁷, Pro³⁸ [Met(O)¹⁴, Trp(O₂)²⁵, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Trp(O₂)²⁵, Asp²⁸]Exendin-4(1-39)-(Lys)₆-NH₂,
H-(Lys)₆- des Pro^{36,} Pro³⁷, Pro³⁸ [Met(O)¹⁴, Trp(O₂)²⁵, Asp²⁸]Exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸] Exendin-4(1-39)-(Lys)₆-NH₂,
oder ein pharmakologisch tolerierbares Salz davon.

Ein weiterer Gegenstand der Erfindung ist eine Formulierung wie oben beschrieben, bei dem zusätzlich Arg³⁴, Lys²⁶ (N^{ε}(γ-glutamyl(N^{α}-hexadecanoyl))) GLP-1 (7-37) [liraglutide] oder ein pharmakologisch tolerierbares Salz davon enthalten ist.

Dem Fachmann ist dabei klar, dass die erfindungsgemäßen Insuline Gegenstand einer pharmazeutischen Formulierung sein können, die nach Applikation vorteilhaft wirkt. Dabei geht man von wässrigen Lösungen aus. Entsprechend müssen weitere Komponenten mischbar sein. Die Gefahr viraler tierischer Kontamination wird dadurch minimiert, dass die Zubereitung keine Komponenten enthalten sollte, die aus tierischen Quellen stammen. Es ist weiterhin vorteilhaft, durch Zusatz von Konservierungsmitteln eine mikrobielle Verunreinigung zu verhindern. Durch den Zusatz isotoner Agentien kann eine mögliche negative Auswirkung der Formulierung auf die Physiologie der Gewebezellen an der Applikationsstelle kompensiert werden. Stabilisierend kann sich der Zusatz von Protamin auswirken, so dass man weitgehend salzfreien Insulinzubereitung gelangen kann, wenn man der Formulierung Protamin zufügt. Der Zusatz von einer phenolischen Komponente kann zu einer Stabilisierung der Struktur des verwendeten Insulinanalogons führen und so unter anderem den Verzögerungseffekt beim Wirkungseintritt zusätzlich bewirken. Der Formulierung zugesetzt können auch Substanzen sein, die die Raumstruktur der erfindungsgemäßen Verzögerungs-insuline stabilisieren und zu besserer thermischen Stabilität führen. Solche chemischen Chaperone können z.B. kurze synthetische Peptide, die auch Aminosäureanaloga enthalten können oder z.B. vom C-Peptid des Insulin abgeleitete Peptidsequenzen umfassen.

Zur Entwicklung von Depotformen können die erfindungsgemäßen Insuline in Nanopartikel eingebunden werden. Denkbar sind auch sogenannte "Slow release" Formuierungen, bei denen das erfindungsgemäße Verzögerungsinsulin reversibel an polymere Träger gebunden vorliegt.

Die erfindungsgemäßen Insuline können parallel zu schnellwirksamen Insulinen wie Apidra^{®}, NovoRapid^{®}, Humalog^{®} oder sich in Entwicklung befindlichen Insulinderivaten oder Formulierungen mit entsprechendem Zeit - / Aktionsprofil oder inhalierbarem Insulin oder nasal oder oral applizierten Insulinen, die sich in Entwicklung befinden, verabreicht werden. Dabei ist es dem Fachmann klar, dass dazu auch entsprechend formulierte Mischungen aus schnell wirksamen und erfindungsgemäßem Verzögerungsinsulin verwendet werden können. Weiterhin können die erfindungsgemäßen Insuinanaloga in pharmazeutischen Zubereitungen verwendet werden, die Peptide, die durch eine dem GLP-1 (Glucagon like Peptide-1) oder dem Exendin-4 bzw. Exendin-3 vergleichbare Aktivität beschrieben sind, enthalten. Beispiele für solche Peptide stellen GLP-1 (7-37), Exenatide (Byetta^{®}) oder Peptide, deren Herstellung in den Patentanmeldungen WO 2006/058620, WO 2001/04156, WO 2004/005342 und WO 98/08871 beschrieben ist, dar. Besonders vorteilhaft sind dabei Formulierungen, die eine Depotformulierung dieser Peptide enthalten. Vorteilhaft besonders in der Anfangsphase der TypII Diabetes Erkrankung sind Therapieformen, die parallel zu Verabreichung der erfindungsgemäßen Pharmazeutika vorsehen, die die Insulinwirkung erhöhen, wie z.B. Metformin. Kombinationstherapien mit Dipeptidyl Peptidase-4 Inhibitoren, die den Spiegel an Inkretinen erhöhen, sind wie Kombinationen mit Sulfonylharnstoffen, die die Insulinausschüttung im Pankreas erhöhen, ebenfalls möglich. Besonders vorteilhaft können die erfindungsgemäßen Verzögerungsinsuline dann eingesetzt werden, wenn durch Applikation von Differenzierungsfaktoren, die Regeneration von Betazellen aus entsprechenden Stammzellen eingeleitet wird. All diese Anwendungen sind beispielhaft für die Therapie des Diabetes genannt und ebenfalls Gegenstand der Erfindung. Ein weiterer Gegenstand der Erfindung ist somit die Verwendung der erfindungsgemäßen Insuline in Kombination mit anderen Wirkstoffen zur Behandlung von Diabetes, insbesondere Diabetes Typ I oder Typ II Diabetes.

Weiterer Erfindungsgegenstand ist auch eine Verwendung der erfindungsgemäßen Insuline bei Regenerationspozessen, die das Skelett, wie z.B. die Knorpelregeneration, betreffen. Bei dieser Verwendung kommt es darauf an, kontrolliert das mitogene Potential von Insulinen zu nutzen, ohne dabei eine starke metabolische Reaktion hervorzurufen.

Ein weiterer Gegenstand der Erfindung ist ein Arzneimittel, das ein erfindungsgemäßes Analogon enthält, welches insbesondere eine wässrige Formulierung oder ein Pulver darstellt.

Das Arzneimittel ist eine pharmazeutische Zubereitung, die vorzugsweise eine Lösung oder Suspension zu Injektionszwecken ist; sie ist gekennzeichnet durch einen Gehalt an mindestens einem erfindungsgemäßen Insulinanalog, und/oder mindestens einem von deren physiologisch verträglichen Salzen in gelöster, amorpher und/oder kristalliner - vorzugsweise in gelöster - Form.

Die Zubereitung weist vorzugsweise einen pH-Wert zwischen etwa 2,5 und 8,5, insbesondere zwischen etwa 4,0 und 8,5 auf, enthält ein geeignetes Isotonisierungsmittel, ein geeignetes Konservierungsmittel und gegebenenfalls einen geeigneten Puffer, sowie vorzugsweise auch eine bestimmte Zink - lonenkonzentration, in steriler wässriger Lösung. Die Gesamtheit der Zubereitungsbestandteile außer dem Wirkstoff bildet den Zubereitungs-Träger. Geeignete Isotonisierungsmittel sind z.B. Glycerin, Glukose, Mannit, NaCl, Calcium- oder Magnesium-Verbindungen wie CaCl₂ etc. Durch die Wahl des Isotonisierungsmittels und/oder Konservierungsstoffes beeinflusst man die Löslichkeit der erfindungsgemäßen Insuline bzw. deren physiologisch verträgliche Salze bei schwach sauren pH-Werten.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 4,0 und 8,5 können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Wenn die Zubereitung einen Zinkgehalt besitzt, ist ein solcher von 1 µg/ml bis 2 mg/ml, insbesondere von 1 µg/ml bis 200 µg Zink/ml bevorzugt. Über den Zusatz von Zink lässt sich das Wirkprofil der erfindungsgemäßen Insulinanaloga überraschend gut beeinflussen Dies erlaubt Zubereitungen, die sich in Bezug auf Gesamtwirkungsdauer, die Geschwindigkeit des Wirkungseintritts und das Profil der Wirkungskurve unterscheiden herzustellen, die so eine individuelle Einstellung des Patienten erlauben.

Zwecks Variation des Wirkstoffprofils der erfindungsgemäßen Zubereitung kann auch unmodifiziertes Insulin, vorzugsweise Rinder-, Schweine- oder Human-Insulin, insbesondere Humaninsulin, oder Insulinanaloga und Derivate davon zugemischt werden. Ebenfalls können ein oder mehrere Exendin-4 Derivate oder Peptide, die durch eine dem GLP-1 (Glucagon like peptide-1) vergleichbare Aktivität charakterisiert sind, zugemischt werden. Solche Arzneimittel (Zubereitungen) sind ebenfalls Gegenstand der Erfindung.

Bevorzugte Wirkstoffkonzentrationen sind solche entsprechend etwa 1 - 1500, weiter bevorzugt etwa 5 - 1000 und insbesondere etwa 40 - 400 internationale Einheiten/ml.

Die erfindungsgemäßen Insulinanaloga werden zunächst als Vorstufe, die noch nicht das Amid umfasst, biotechnologisch hergestellt. Dem Fachmann ist geläufig, das es eine Vielzahl von Möglichkeiten zur Herstellung von Insulinen gibt. Als Wirtszellsysteme finden dabei Bakterien, Hefen und höhere Pflanzen bzw. fermentativ zu kultivierende Pflanzenzellen Verwendung. Falls die Kostenbetrachtung es erlaubt sind auch Expressionssysteme, die tierische Zellen als Wirtssystem nutzen, denkbar. Voraussetzung dafür ist aber eine sichere Freiheit von tierischen Viren. Somit ist klar, dass die beispielhaft beschriebenen Expressionssysteme nur einen kleinen Ausschnitt der für die rekombinante Herstellung von Proteinen entwickelten Wirts/Vektorsysteme darstellen. In der Anmeldung werden z.B. biotechnologische Verfahren, die Hefe- oder Pflanzensysteme wie Moose, Algen oder höhere Pflanzen wie Tabak, Erbse, Distel, Gerste, Mais oder Raps zur Grundlage haben nicht beschrieben. Wirts/Vektor Systeme sowie kodierende DNA- Sequenzen, die die Herstellung der Zielpeptide in entsprechenden biotechnologischen Expressionssystemen erlauben, werden beschrieben. Wirtsorganismen können also insbesondere ausgewählt werden aus dem Pflanzenreich aus Organismen der ersten Abteilung Schizophyta enthaltend Schizomycetes, Bakterien oder Blaualgen , Organismen der 2. Abteilung Phycophyta V. Klasse Chlorophyceae, Organismen der 2. Abteilung Phycophyta VII. Klasse Rhodophyceae, Organismen der 3. Abteilung Mycophyta, Organismen der 5. Abteilung Bryophyta und Organismen der 7. Abteilung Spermatophyta.

In der Europäischen Patentanmeldung EP-A 1 222 207 ist ein Plasmid pINT358d beschrieben, das für ein Prä - Proinsulin codiert, welches ein verändertes C-Peptid umfasst. Mit Hilfe der Polymerase Kettenreaktion (PCR) ist es nun möglich, gezielt die das Proinsulin kodierende Sequenz so zu verändern, dass Prä - Proinsuline exprimiert werden können, die als Vorstufen für die erfindungsgemäßen Insuline dienen können. Entsprechende Fusionsproteine müssen nicht notwendigerweise intrazellulär hergestellt werden. Es ist dem Fachmann klar, dass solche Proteine auch durch bakterielle Expression mit anschließender Sekretion in das Periplasma und/oder in den Kulturüberstand hergestellt werden können. Die Europäische Patentanmeldung EP-A 1 364 029 beschreibt dies beispielhaft.

Die so hergestellten Proinsuline können prinzipiell zu einer Insulinanalogavorstufe umgewandelt werden, die in Position A0 Lysin oder Arginin umfasst und am C-terminalen Ende der B - Kette Lysin oder Arginin trägt. Alternativ kann eine positiv geladene Aminosäure semisynthetisch an den N-Terminus derA-Kette addiert werden, wenn Lysin oder Arginin nicht ohnehin schon vorliegen.

Liegen die Proinsuline nach intrazellulärer Expression in Bakterien als Einschlusskörper oder löslich vor, müssen diese Vorstufen durch in vitro Faltung in die richtige Konformation gefaltet werden, bevor die Prozessierung und biochemische Modifikation vorgenommen werden kann. Dabei erlaubt das beschriebene Fusionsprotein eine direkte Faltung nach Denaturierung mittels Harnstoff oder Guanidinium Hydrochlorid.

Zur Anreicherung der einzelnen Zwischenstufen finden biochemische Methoden insbesondere Trennverfahren Verwendung, deren zugrunde liegenden Prinzipien publiziert und sogar Gegenstand von Lehrbüchern sind. Dem Fachmann ist klar, dass solche Prinzipien in Folge kombiniert werden können und so zu Verfahren führen können, die in ihre Abfolge vorher nicht publiziert wurden.

Ein weiterer Gegenstand der Erfindung ist ein Verfahren zur Herstellung der erfindungsgemäßen Insulinanaloga, wobei ein Vorläufer des Insulinanalogons rekombinant hergestellt und enzymatisch zu einer 2 - kettigen Insulinvorstufe umgewandelt wird, die N-terminal zu Aminosäure 1 der A- Kette Arginin bzw. Lysin trägt und am C-terminalen Ende der B-Kette einen Lysin oder Argininrest aufweist, der mit Argininamid oder Lysinamid in Gegenwart eines Enzyms mit Trypsinaktivität in das Amid und somit in das erfindungsgemäße Verzögerungsinsulin überführt und über ein biochemischer Reinigungsverfahren hochrein dargestellt wird.

Proteine, welche sich durch Substitution wenigstens eines natürlich auftretenden Aminosäurerestes mit anderen Aminosäureresten und/oder Addition und/oder Entfernen wenigstens eines Aminosäurerestes von dem entsprechenden, ansonsten gleichen natürlich vorkommenden Protein unterscheiden, werden als "Analoga" von Proteinen bezeichnet. Dabei kann es sich bei den hinzugefügten und / oder ersetzten Aminosäureresten auch um solche handeln, die nicht natürlich vorkommen.

Proteine, welche durch chemische Modifizierung bestimmter Aminosäurereste von Ausgangsproteinen erhalten werden, bezeichnet man als "Derivate" von Proteinen. Die chemische Modifikation kann z.B. in der Addition einer oder mehrerer bestimmter chemischer Gruppen an eine oder mehrere Aminosäuren bestehen.

Figurenlegende:
- Fig. 1 :: Blutzuckersenkende Wirkung von erfindungsgemäßen Insulinanaloga in Ratten
- Fig. 2:: Blutzuckersenkende Wirkung von Insulin Glargin in Ratten

### Beispiel 1 : Herstellung des Vektorderivates pINT3580, das für Gly (A21) - Insulin und ein modifiziertes C-Peptid, das an der C/A- Kettengrenze Arg Arg trägt, kodiert.

Die Europäische Patentanmeldung EP-A 1 222 207 beschreibt die Plasmide pINT358d, pINT91d und die Primer - Sequenz Tir. DNA dieser Produkte findet Verwendung in der Konstruktion des Plasmides pINT3580. Das Plasmid pINT358d ist dabei durch eine Gensequenz charakterisiert, die für ein modifiziertes C-Peptid mit besonderen Eigenschaften kodiert. Drei Primersequenzen werden synthetisiert:
pint3580_glya21 rev
   5'- CAAAGGTCGACTATTA**GCC**GCAGTAGTTCTCCAGCTGG-3' (SEQ ID NO: 3)

Dieser Primer dient nach Aufarbeitung dazu in Position 21 der A-Kette der von pINT358d kodierten Proinsulinsequenz Glycin (fett gedruckt, unterstrichen) anstelle von Asparagin einzuführen.
arg_cjuncf
   5'-GTCCCTGCAGCGTCGCGGCATCGTGGAGCAG-3' (SEQ ID NO: 4)

Dieser Primer dient, wie der Primer arg_cjunc_rev, zur Einführung von Arginin anstelle von Lysin an der Insulin A-/ B- Kettengrenze.
arg_cjunc_rev
   5'- CCACGATGCC GCG**ACG**CTGC AGGGACCCCT CCAGCG-3' (SEQ ID NO: 5)

Das Codon für das einzuführende Arginin ist in beiden Primern fett gedruckt. Mit DNA des Plasmides pINT358d als Matrize wird mit den Primerpaaren Tir / arg_cjunc_rev und arg_cjuncf / pint3580_glya21rev entsprechend der Europäischen Patentanmeldung EP-A 1 222 207 je eine PCR durchgeführt. Aliquots der Produkte beider Reaktionen werden kombiniert und zusammen mit dem Primerpaar Tir / pint3580_glya21rev in einer dritten PCR eingesetzt. Das Produkt dieser Reaktion wird nach gelektrophoretischer Auftrennung des Reaktionsgemisches gereinigt und mit den Restriktionsenzymen Sal1 / Nco1 nach Angaben des Herstellers in ein und derselben Reaktion verdaut, das Reaktionsgemisch gelelektrophoretisch aufgetrennt und das die Proinsulinsequenz kodierende DNA - Fragment isoliert. Das Fragment wird anschließend über eine DNA- Ligasereaktion in die Nco1 / Sal1 geöffnete pINT91d Vektor DNA insertiert.

Mit dem Ligationsgemisch werden kompetente E. coli Bakterienzellen transformiert. Das Transformationsgemisch wird auf Selektionsplatten, die 25 mg/l Ampicillin enthalten, ausplattiert. Plasmid DNA wird von Kolonien isoliert und mittels DNA - Sequenzanalyse charakterisiert. Richtige Plasmide erhalten die Bezeichnung pINT3580.

### Beispiel 2: Konstruktion des Plasmides pINT3581 kodierend für His (A8), Gly (A21) - Präproinsulin

Die Konstruktion erfolgt wie in Beispiel 1 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3580_Ha8f
   5'-AGCAGTGCTGC**CAC**AGCATCTGCTCCCTCTAC-3' (SEQ ID NO: 6)
pint3580_Ha8rev
   5'-GAG CAGATGCT **GTG** GCAGCACTG CTCCACGATG-3'(SEQ ID NO: 7)

Das Codon, das für Histidin in Position 8 der A- Kette kodiert ist jeweils fett herausgestellt. Die Konstruktion wird wie ins Beispiel 1 beschrieben durchgeführt. Template für PCR1 und 2 ist DNA des Plasmides pINT3580. PCR1 wird mit dem Primerpaar Tir/ pint3580_Ha8rev und PCR2 mit dem Primerpaar pint3580_Ha8f/ pint3580_glya21rev durchgeführt. In PCR3 wird mit dem Primerpaar Tir/ pint3580_glya21rev eingesetzt. Template ist dabei ein Gemisch der Reaktionsprodukte von PCR1 und PCR2. Richtige Plasmide erhalten die Bezeichnung pINT3581.

Das von dem Plasmid kodierte Prä - Proinsulin ist Vorstufe für die Verbindung YKL202, die als Arg (A0), His (A8), Gly (A21), Arg (B31), Arg (B32) - NH₂ - Humaninsulin charakterisiert ist und die nach Amidierung mit Argininamid entsteht. Das von dem Plasmid kodierte Prä - Proinsulin ist Vorstufe für die Verbindung YKL202b, die als Arg (A0), His (A8), Gly (A21), Arg (831), Lys (B32) - NH₂ - Humaninsulin charakterisiert ist und die nach Amidierung mit Lysinamid entsteht.

### Vergleichsbeispiel 3: Konstruktion des Plasmides pINT3582 kodierend für His (A8), Glu (A5), Gly (A21) - Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3581_Ea5f
   5'GCATCGTGGAG**GAG**TGCTGCCACAGCATCTG 3' (SEQ ID NO: 8)
pint3581_Ea5rev
   5'-CTGT GGCAGCA**CTC** CTCCACGATG CCGCGACG-3' (SEQ ID NO: 9)

Das Codon, das für Glutaminsäure in Position 5 der A- Kette kodiert, ist jeweils fett herausgestellt. Die Konstruktion wird wie ins Beispiel 1 beschrieben durchgeführt. Template ist DNA des Plasmides pINT3581. Richtige Plasmide erhalten die Bezeichnung pINT3582.

Das von dem Plasmid kodierte Prä - Proinsulin ist Vorstufe für die Verbindung YKL202-1, die als Arg (A0), Glu (A5), His (A8), Gly (A21), Arg (B31), Arg (B32) - NH₂ - Humaninsulin charakterisiert ist und die nach Amidierung mit Argininamid entsteht.

Das von dem Plasmid kodierte Prä - Proinsulin ist Vorstufe für die Verbindung YKL202-1b, die als Arg (A0), Glu (A5), His (A8), Gly (A21), Arg (B31), Lys (B32) - NH₂ - Humaninsulin charakterisiert ist und die nach Amidierung mit Lysinamid entsteht.

### Vergleichsbeispiel 4: Konstruktion des Plasmides pINT3583 kodierend für His (A8), Asp (A18), Gly(A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. Ein weiterer Primer wird synthetisiert:
pint3580_Da18rev
   5'CAAAGGTCGACTATTAGCCGCAGTA**GTG**CTCCAGCTGGTAGAGGGAG 3' (SEQ ID NO: 10)

Das Codon, das für Asparaginsäure in Position 18 der A- Kette kodiert, ist fett herausgestellt. Template ist DNA des Plasmides pINT3581. Richtige Plasmide erhalten die Bezeichnung pINT3583.

Das von dem Plasmid kodierte Prä - Proinsulin ist Vorstufe für die Verbindung YKL202, die als Arg (A0), Asp (A18), His (A8), Gly (A21), Arg (B31), Arg (832) - NH₂ - Humaninsulin charakterisiert ist und die nach Amidierung mit Argininamid entsteht.

Das von dem Plasmid kodierte Prä - Proinsulin ist Vorstufe für die Verbindung YKL202-2b, die als Arg (A0), Asp (A18), His (A8), Gly (A21), Arg (B31), Lys (832) - NH₂ - Humaninsulin charakterisiert ist und die nach Amidierung mit Lysinamid entsteht.

### Beispiel 5: Konstruktion des Plasmides pINT3585 kodierend für His (A8), Glu (A15), Gly (A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. Ein weiterer Primer wird synthetisiert:
pint3580_Ea15rev

Das Codon, das für Glutaminsäure in Position 15 der A- Kette kodiert, ist fett herausgestellt. Template ist DNA des Plasmides pINT3581. Richtige Plasmide erhalten die Bezeichnung pINT3585.

Das von dem Plasmid kodierte Prä - Proinsulin ist Vorstufe für die Verbindung YKL202-3, die als Arg (A0), Glu (A15), His (A8), Gly (A21), Arg (B31), Arg (B32) - NH₂ - Humaninsulin charakterisiert ist und die nach Amidierung mit Argininamid entsteht.

Das von dem Plasmid kodierte Prä - Proinsulin ist Vorstufe für die Verbindung YKL202-3b, die als Arg (A0), Glu (A15), His (A8), Gly (A21), Arg (B31), Lys (B32) - NH₂ - Humaninsulin charakterisiert ist und die nach Amidierung mit Lysinamid entsteht.

### Vergleichsbeispiel 6: Konstruktion des Plasmides pINT3588 kodierend für His (A8), Gly (A21), Asp (B3)- Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
5' - GCACGATTTGTG**GAC**CAGCACCTGTGCGGC -3' (SEQ ID NO: 12)
pint3581_Db3rev
   5' - CACAGG TGCT**GGT**CCA CAAATCGTGC CGAATTTC -3' (SEQ ID NO: 13)

Das Codon, das für Asparagin in Position 3 der Insulin B- Kette kodiert ist jeweils fett herausgestellt. Die Konstruktion wird wie ins Beispiel 1 beschrieben durchgeführt. Template ist DNA des Plasmides pINT3581. Richtige Plasmide erhalten die Bezeichnung pINT3588.

Das von dem Plasmid kodierte Prä - Proinsulin ist Vorstufe für die Verbindung YKL202-4, die als Arg (A0), His (A8), Gly (A21), Asp (B3), Arg (B31), Arg (B32) - NH₂ - Humaninsulin charakterisiert ist und die nach Amidierung mit Argininamid entsteht.

Das von dem Plasmid kodierte Prä - Proinsulin ist Vorstufe für die Verbindung YKL202-4b, die als Arg (A0), His (A8), Gly (A21), Asp (B3), Arg (B31), Lys (B32) - NH₂ - Humaninsulin charakterisiert ist und die nach Amidierung mit Lysinamid entsteht.

### Vergleichsbeispiel 7: Konstruktion des Plasmides pINT3593 kodierend für His (A8), Gly (A21), Glu (B4) Präproinsulin

Die Konstruktion erfolgt wie in Beispiel 1 und 2 beschrieben über 3 Polymerase Kettenreaktionen Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert :
pint3581_Eb4f
   5' - ACGATTTGTGAAC**GAG**CACCTGTGCGGCTC -3' (SEQ ID NO: 14)
pint3581_Eb4rev
   5'- CGCACAGG TG**CTC**CTTCA CAAATCGTGC CGAATTTC -3' (SEQ ID NO: 15)

Das Codon, das für Glutamin in Position 4 der Insulin B- Kette kodiert, ist fett herausgestellt. Die Konstruktion wird wie ins Beispiel 1 beschrieben durchgeführt. Template ist DNA des Plasmides pINT3581. Richtige Plasmide erhalten die Bezeichnung pINT3593.

Das von dem Plasmid kodierte Prä - Proinsulin ist Vorstufe für die Verbindung YKL202-5, die als Arg (A0), His (A8), Gly (A21), Glu (B4), Arg (B31), Arg (B32) - NH₂- Humaninsulin charakterisiert ist und die nach Amidierung mit Argfninamid entsteht.

Das von dem Plasmid kodierte Prä - Proinsulin ist Vorstufe für die Verbindung YKL202-5b, die als Arg (A0), His (A8), Gly (A21), Glu (B4), Arg (B31), Lys (B32) - NH₂ - Humaninsulin charakterisiert ist und die nach Amidierung mit Lysinamid entsteht.

### Vergleichsbeispiel 8: Konstruktion des Plasmides pINT3597 kodierend für His (A8), Gly (A21), Glu (B0) -Präproinsulin

Die Konstruktion erfolgt über 2 Polymerase Kettenreaktionen. Verwendet wird der Primer pint3580_glya21 rev. Zwei weitere Primer werden synthetisiert:
pint3581_Eb0f1
   5' - CAACAGGAAATTCGGCACG A**GAG**TTTGTG AACCAGCACC TGTG-3' (SEQ ID NO: 16)
pint3581_Eb01f2
   5'- TATCGA CCAT GG CAACAACA TCAACAGGAAATTCGGCACG A**GAG**-3' (SEQ ID NO: 17)

Dabei überlappen sich die beiden Primer partial. Pint3581_Eb0f2 enthält eine NcoI - Erkennungssequenz. Diese ist unterstrichen dargestellt. Das Codon, das für Glutaminsäure in Position 0 am Anfang B- Kette kodiert, ist jeweils fett herausgestellt. Template für PCR1 ist DNA des Plasmides pINT3581.

PCR1 wird mit dem Primerpaar pint3581_Eb-1f2 / pint3580_glya21rev. Template für PCR2 ist das Produkt aus PCR1. PCR2 wird mit dem Primerpaar pint3581_Eb-1f2 / pint3580_glya21rev durchgeführt. Das Produkt aus PCR2 übereckt die vollständige Präproinsulinsequenz. Das Produkt der zweiten Reaktion wird nach Nco1/ Sal1 Spaltung in die Nco1 / Sal1 geöffnete pINT91d Vektor DNA insertiert. Richtige Plasmide erhalten die Bezeichnung pINT3597.

Das von dem Plasmid kodierte Prä - Proinsulin ist Vorstufe für die Verbindung YKL202-6, die als Arg (A0), His (A8), Gly (A21), Glu (B0), Arg (B31), Arg (B32) - NH₂ - Humaninsulin charakterisiert ist und die nach Amidierung mit Argininamid entsteht.

Das von dem Plasmid kodierte Prä - Proinsulin ist Vorstufe für die Verbindung YKL202-6b, die als Arg (A0), His (A8), Gly (A21), Glu (B0), Arg (B31), Lys (B32) - NH₂ - Humaninsulin charakterisiert ist und die nach Amidierung mit Lysinamid entsteht.

Ersetzt man das Codon für Glutaminsäure in Position B0 durch das Codon von Asparaginsäure und folgt dem Beispiel, so gelangt man zu Plasmiden, die in Position B0 anstelle von Glutaminsäure Asparaginsäure tragen.

### Vergleichsbeispiel 9: Konstruktion des Plasmides pINT3700 kodierend für His (A8), Gly (A21), Lys (B0) - Präproinsulin

Die Konstruktion dient dazu, an der Grenze zwischen Präsequenz und Start der B-Kette anstelle des Codons für Arginin das Codon für Lysin einzuführen. Die Konstruktion erfolgt über zwei Polymerase Kettenreaktionen. Verwendet wird der Primer pint3580_glya21rev. Zwei weitere Primer werden synthetisiert.
pint3581_Eb0f1
   5'- CAACAGGAA ATTCGGCA **AAG**TTTGTG AACCAGCACC TGTG - 3' (SEQ ID NO: 18)
pint3581_Eb01f2
   5'- TATCGA CCAT GG CAACAACA TCAACAGGAA ATTCGGCA**GAG** - 3' (SEQ ID NO: 19)

Dabei überlappen sich die beiden Primer partial und pint3581_Eb0f2 enthält eine NcoI - Erkennungssequenz. Diese ist unterstrichen dargestellt. Das Codon, das für Glutaminsäure in Position 0 am Anfang B- Kette kodiert, ist jeweils fett herausgestellt. Template für PCR1 ist DNA des Plasmides pINT3581. PCR1 wird mit dem Primerpaar pint3581_Eb-1f2 / pint3580_glya21rev durchgeführt. Template für PCR2 ist das Produkt aus PCR1. PCR2 wird mit dem Primerpaar pint3581_Eb-1f2 / pint3580_glya21rev durchgeführt. Das Produkt aus PCR2 überdeckt die vollständige Präproinsulinsequenz. Das Produkt der zweiten Reaktion wird nach Nco1/ Sal1 Spaltung in die Nco1 / Sal1 geöffnete pINT91d Vektor DNA insertiert. Richtige Plasmide erhalten die Bezeichnung pINT3700.

Das von dem Plasmid kodierte Prä - Proinsulin ist Vorstufe für die Verbindung Arg (A0), His (A8), Gly (A21), Arg (B30) - NH₂ - Humaninsulin. Um zu der Verbindung zu gelangen stellt man zunächst durch Umsetzung mit Trypsin und Lysyl - Endopeptidase C die Zwischenstufe Arg (A0), His (A8), Gly (A21), des Thr (B30) - Humaninsulin her, die anschließend über Amidierung mit Argininamid zu der gewünschten Verbindung YKL202-7, die als Arg (A0), His (A8), Gly (A21), Arg (B30) - NH₂ - Humaninsulin charakterisiert ist, umgewandelt wird.

Das von dem Plasmid kodierte Prä - Proinsulin ist Vorstufe für die Verbindung YKL202-7b, die als Arg (A0), His (A8), Gly (A21), Lys (B30) - NH2 - Humaninsulin charakterisiert ist und die nach Amidierung mit Lysinamid entsteht.

### Vergleichsbeispiel 10: Konstruktion des Plasmides pINT3701 kodierend für Gly (A21) Lys (B0) - Präproinsulin

Die Konstruktion dient dazu, an der Grenze zwischen Präsequenz und Start der B-Kette anstelle des Codons für Arginin das Codon für Lysin einzuführen. Folgt man der Konstruktionsstrategie aus Vergleichsbeispiel 9, verwendet aber als Template für PCR1 DNAdes Plasmides pINT358d, so gelangt man zu dem Plasmid pINT3701, das wie das ursprüngliche Plasmid pINT358d dadurch charakterisiert ist, das die Grenze zwischen C- Peptid und A- Kette durch die Aminosäurefolge Lys-Arg gebildet wird

### Vergleichsbeispiel 11: Konstruktion des Plasmides pINT3702 kodierend für His (A8), Gly (A21), Lys (B0) - Präproinsulin mit dem C- Peptid gemäß Vergleichsbeispiel 10

Folgt man dem im Beispiel 2 beschriebenen Konstruktionsplan, verwendet aber als Template DNA des Plasmides pINT3701, so gelangt man zu dem Plasmid pINT3702.

Das von dem Plasmid kodierte Prä - Proinsulin ist Vorstufe für die Verbindung Arg (A0), His (A8), Gly (A21), Arg (B30) - NH₂ -Humaninsulin. Um zu der Verbindung zu gelangen stellt man zunächst durch Umsetzung nur mit Lysyl - Endopeptidase C die Zwischenstufe Arg (A0), His (A8), Gly (A21), des Thr (B30) - Humaninsulin her, die anschließend über Amidierung mit Argininamid zu der gewünschten Verbindung YKL202-8, die als Arg (A0), His (A8), Gly (A21), Arg (B30) - NH2 - Humaninsulin charakterisiert ist, umgewandelt wird

Das von dem Plasmid kodierte Prä - Proinsulin ist Vorstufe für die Verbindung YKL202-8b, die als Arg (A0), His (A8), Gly (A21), Lys (B30) - NH₂ - Humaninsulin charakterisiert ist und die nach Amidierung mit Lysinamid entseht. Vergleichsbeispiel 12: Herstellung des Vektorderivates pINT3703, das für His (A8), Gly (A21), Lys (B0) - Präproinsulin und ein modifiziertes C-Peptid, das an der C/A-Kettengrenze das Tri-Peptid Lys-Arg-Arg trägt, kodiert.

Benutzt wird DNA des Plasmides pINT3702 als Template, sowie die Primer pint3580_glya21 rev und Tir. Zwei Primersequenzen werden synthetisiert.
3702_arg_cjuncf
   5' - TGC AGAAGCGCAGAGGCATCGTG GAGCAGTGC - 3' (SEQ ID NO: 20)

Dieser Primer dient wie der Primer arg_cjunc_rev zur Einführung von Arginin an der Insulin A-/ B- Kettengrenze.
3702_cjunc_rev
   5' - TCC ACGATGCC**TCT**GCGCTTCTG CAGGGACCC - 3' (SEQ ID NO: 21)

Das Codon für das einzuführende Arginin ist in beiden Primern fett gedruckt. Mit DNA des Plasmides pINT3702 als Matrize werden mit den Primerpaaren Tir / 3702_cjunc_rev und 3702_arg_cjuncf / pint3580_glya21 rev entsprechend EP-A1 222 207 je eine PCR durchgeführt. Aliquots der Produkte beider Reaktionen werden kombiniert und zusammen mit dem Primerpaar Tir / pint3580_glya21rev in einer dritten PCR eingesetzt. Das Produkt dieser Reaktion wird nach gelektrophoretischer Auftrennung des Reaktionsgemisches gereinigt und mit den Restriktionsenzymen Sal1 / Nco1 nach Angaben des Herstellers in ein und derselben Reaktion verdaut, das Reaktionsgemisch gelelektrophoretisch aufgetrennt und das die Proinsulinsequenz kodierende DNA - Fragment isoliert. Das Fragment wird anschließend über eine DNA- Ligasereaktion in die Nco1 / Sal1 geöffnete pINT91d Vektor DNA insertiert.

Mit dem Ligationsgemisch werden kompetente E. coli Bakterienzellen transformiert. Das Transformationsgemisch wird auf Selektionsplatten, die 25 mg/l Ampicillin enthalten ausplattiert. Plasmid DNA wird von Kolonien isoliert und mittels DNA - Sequenzanalyse charakterisiert. Richtige Plasmide erhalten die Bezeichnung pINT3702.

Das von dem Plasmid kodierte Prä - Proinsulin ist Vorstufe für die Verbindung Arg (A-1), Arg (A0), His (A8), Gly (A21), Arg (B30) - NH₂ - Humaninsulin. Um zu der Verbindung zu gelangen, stellt man zunächst durch Umsetzung mit Lysyl - Endopeptidase C die Zwischenstufe Arg (A-1), Arg (A0), His (A8), Gly (A21), des Thr (B30) - Humaninsulin her, die anschließend über Amidierung mit Argininamid zu der gewünschten Verbindung umgewandelt wird.

Das von dem Plasmid kodierte Prä - Proinsulin ist Vorstufe für die Verbindung Arg (A-1), Arg (A0), His (A8), Gly (A21), Lys (B30) - NH₂ - Humaninsulin, die nach Amidierung mit Lysinamid entsteht.

Es ist dem Fachmann klar, dass man entsprechend den Beispielen 3-8 Asparaginsäure oder Glutaminsäure in die entsprechenden Positionen der A bzw. B- Kette einführen kann. Diese Proinsuline sind Vorstufe für die Verbindungen

| | |
|---|---|
| YKL202-10a | Arg (A-1), Arg (A0), Glu (A5), His (A8), Gly (A21), Arg (B30) - NH₂ - Humaninsulin |
| YKL202-10b | Arg (A-1), Arg (A0), Glu (A5), His (A8), Gly (A21), Lys (B30) - NH₂ - Humaninsulin |
| YKL202-11a | Arg (A-1), Arg (A0), Glu (A15), His (A8), Gly (A21), Arg (B30) - NH₂ - Humaninsulin |
| YKL202-11b | Arg (A-1), Arg (A0), Glu (A15), His (A8), Gly (A21), Lys (B30) - NH₂ - Humaninsulin |
| YKL202-12a | Arg (A-1), Arg (A0), Asp (A18), His (A8), Gly (A21), Arg (B30) - NH₂ - Humaninsulin |
| YKL202-12b | Arg (A-1), Arg (A0), Asp (A18), His (A8), Gly (A21), Arg (B30) - NH₂ - Humaninsulin |
| YKL202-13a | Arg (A-1), Arg (A0), His (A8), Gly (A21), Glu (B0), Arg (B30) - NH₂ - Humaninsulin |
| YKL202-13b | Arg (A-1), Arg (A0), His (A8), Gly (A21), Glu (B0), Lys (B30) - NH₂ - Humaninsulin |
| YKL202-14a | Arg (A-1), Arg (A0), His (A8), Gly (A21), Asp (B3), Arg (B30) - NH₂-Humaninsulin |
| YKL202-14b | Arg (A-1), Arg (A0), His (A8), Gly (A21), Asp (B3), Lys (B30) - NH₂-Humaninsulin |
| YKL202-15a | Arg (A-1), Arg (A0), His (A8), Gly (A21), Glu (B4), Arg (B30) - NH₂ - Humaninsulin |
| YKL202-15b | Arg (A-1), Arg (A0), His (A8), Gly (A21), Glu (B4), Lys (B30) - NH₂ - Humaninsulin |

### Vergleichsbeispiel13: Herstellung des Vektorderivates pINT3704, das für His (A8), Gly (A21), Lys (B0) - Präproinsulin kodiert

Dieses Beispiel beschreibt die Herstellung des Vektorderivates pINT3704, das für His (A8), Gly (A21), Lys (B0) - Präproinsulin kodiert und ein modifiziertes C-Peptid, das an der C/A- Kettengrenze das Tri-Peptid Lys-Arg-Arg trägt, und bei dem die Aminosäure Glycin, welche in Humaninsulin in Position A21 vorkommt, deletiert ist.

Folgt man dem in Vergleichsbeispiel 12 beschrieben Syntheseschema und ersetzt die Primer 3702_arg_cjuncf und 3702_arg_cjuncfrev durch die Primer 3703_Δ Ga1f und 3703_Δ Ga1rev und benutzt DNA des Plasmides pINT3703 als Template, so gelangt man zu dem Plasmid pINT3704, das dadurch gekennzeichnet ist, das das in dem kodierten Präproinsulin die Aminosäure Glycin in Position1 der A- Kette deletiert ist und der übrige Sequenzverlauf dem, der von pINT3703 kodierten Sequenz entspricht.
3703_Δ Ga1f
   5' - TGC AGAAGCGC**AGA**ATCGTG GAGCAGTGCTGC - 3' (SEQ ID NO: 22)
3703_Δ Ga1rev
   5' - TGCTCC ACGATTCTGCGCTTCTG CAGGGACCC - 3' (SEQ ID NO: 23)

Das von dem Plasmid kodierte Prä - Proinsulin ist Vorstufe für die Verbindung Arg (A0), Arg (A1), His (A8), Gly (A21), Arg (B30) - NH₂ - Humaninsulin. Um zu der Verbindung zu gelangen, stellt man zunächst durch Umsetzung mit Lysyl-Endopeptidase C die Zwischenstufe Arg (A0), Arg (A1), His (A8), Gly (A21), des Thr (B30) - Humaninsulin her, die anschließend über Amidierung mit Argininamid zu der gewünschten Verbindung umgewandelt wird.

Das von dem Plasmid kodierte Prä - Proinsulin ist Vorstufe für die Verbindung Arg (A0), Arg (A1), His (A8), Gly (A21), Lys (B30) - NH₂ - Humaninsulin, die nach Amidierung mit Lysinamid entsteht.

Dem Fachmann ist klar, dass man entsprechend Beispiel 12 negativ geladene Aminosäuren in die Präproinsulinsequenz einführen kann. Auch lässt sich in Analogie zu Beispiel 13 aus dem Plasmid pINT3702 ein Plamid herstellen, dass für eine Proinsulinsequenz kodiert, die die Herstellung von Insulinderivaten erlaubt, die in Position 1 der A-Kette ein Arginin als N- terminale Aminosäure tragen.

### Beispiel 14: Expression der Proinsulinderivate

Die Expression wird entsprechend Beispiel 1 der Europäischen Patentanmeldung EP-A 1 222 207 durchgeführt.

### Beispiel 15: Faltung der Proinsulinderivate

Die Faltung erfolgt prinzipiell nach der in EP-A 0 668 282 beschriebenen Methode.

### Beispiel 16: Prozessierung der Proinsuline aus den Beispielen 2-8 mittels Trypsin

Es erfolgt die enzymatische Prozessierung der gefalteten Präproinsulin zu der zweikettigen Arg (A0) -Insulinvorstufe, deren C-terminales B-Kettenende durch Lysin oder Arginin charakterisiert ist. Die enzymatische Prozessierung der gefalteten Präproinsulinvorstufe erfolgt wie z.B. in Beispiel 4 von WO91/03550 beschrieben. Als besonders vorteilhaft erweist sich dabei der Einsatz der in WO 2007/031187 A1 beschriebenen Trypsinvariante.

### Vergleichsbeispiel 17: Prozessierung der Proinsuline aus den Beispielen 9-13 mittels Endoproteinase Lys-C

Endoprotease Lys-C aus Achromobacter lyticus ist kommerziell erhältlich (Merck/Calbiochem). Die Reaktion wird leicht modifiziert wie von Jekel, P.A. et al. [Anal. Biochem. 134, 347-354,(1983)] beschrieben durchgeführt. Es wird ein pH von 9,5 eingestellt und die Reaktionstemperatur beträgt 30°C.

In der Eintopfreaktion wir die Präsequenz abgespalten und das C-Peptid beginnend mit Thr (B30) herausgespalten, so dass das C-terminale Ende der B- Kette von Lysin gebildet wird, das als reaktive Stelle für die enzymkatalysierte Kupplungsreaktion vorliegt.

### Beispiel 18: Herstellung der Arg - NH₂ bzw. Lys - NH₂ Insulinverbindung aus der zweikettigen Vorstufe über Kupplung mit Arginin - bzw. Lysinamid

Unabhängig von der Positionierung der zusätzlichen sauren Aminosäuren wird eine Standardreaktion wie folgt durchgeführt: 100 mg Arg (A0), Gly (A21), Arg (B31)-Insulinanalog werden in 0.95 ml Argininamidlösung (446 g/L) gelöst , 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet. Nach 8 h wird die Reaktion durch Zugabe von TFA bis pH 2.5 gestoppt und per HPLC analysiert. Es bildet sich >60 % . Arg (A0), Gly (A21), Arg (B31), Arg (B32)- NH₂- Insulinanalogon Nach Zusatz von Trypsininhibitorlösung erfolgt die Reinigung des amidierten Analogs in Analogie zu US 5,656,722.

Die Herstellung der entsprechenden Lysinamid - Verbindung erfolgt analog. Allerdings geht man von einer wässrigen Lysinamid Stammlösung aus, die 366g/L Lysinamid gelöst enthält.

### Vergleichsbeispiel 19: Semisynthetische Herstellung von Lys (A-1), Arg (A0), His (A8), Gly (A21), Arg (B30) - Insulinamid

Die in Beispiel 11 beschriebene Verbindung YKL202-8, die einem Insulinderivat der Struktur Arg (A0), His (A8), Gly (A21), Arg (B30) - Insulinamid entspricht, dient als Ausgangsmaterial zur Herstellung der Verbindung YKL202-11, die Lys (A-1), Arg (A0), His (A8), Gly (A21), Arg (B30) - Insulinamid. 200mg der Verbindung werden in 40 ml eines 100mM Na₂ HPO₄ / CH₃CN (50:50) - Gemisches gelöst und ein pH von 7,7 - 8,2 eingestellt. Als weiteres Material wird ein Boc-Lys (Boc) - NHS Ester hergestellt indem man 0,3 mM Boc-Lys(Boc)-OH, 0,4 mM N-hydroxysuccineimide (NHS) und 0,4mM Dicyclohexylcarbodiimid (DCC) in Dichlomnethan 40 Minuten mischt. Das Reaktionsgemisch im Rotationsvakuumverdampfer zur Trockene eingeengt. Das Gemisch wird anschließend in 5 ml Methanol aufgenommen und dem gelösten Ausgangsinsulin zugesetzt. Die Reaktion wird bei Raumtemperatur mindestens 60, maximal aber 120 Minuten gerührt. Die Reaktion wird durch Zugabe von 200µl TFA gestoppt. Die Masse der Reaktionsprodukte wird über LC-MS massenspektroskopisch charakterisiert, um den Nachweis zu erbringen, dass monoacyliertes Produkt, das aus drei Komponenten besteht vorliegt. Die Komponenten werden über HPLC getrennt. Dabei werden auch di- acylierte und triacylierte Nebenprodukte abgetrennt. Die Fraktionen der HPLC - Trennung werden massenspektrokopisch analysiert. Die 3 Fraktionen , in denen jeweils monoacyliertes Produkt enthalten, werden einer Aminosäuresequenzanalyse unterzogen, deren Interpretation die Identifikation der HPLC - Fraktion erlaubt, die die gewünschte Verbindung enthält von der anschließend durch Hydrolyse die Boc-Schutzgruppen entfernt werden. Nach erneuter HPLC- Reinigung wird das gewünschte Produkt auf seine biologischen Eigenschaften getestet.

### Beispiel 20: Herstellung des Plasmides pINT358_ha8, das für His (A8), Gly (A21) Proinsulin kodiert.

Folgt man dem Herstellungsschema aus Beispiel 2 und verwendet als Template für die PCR1 und PCR2 DNA des Plasmides pINT358d, so gelangt man zu dem Plasmid pINT358_ha8, das für His (A8), Gly (A21) Proinsulin kodiert. Das von dem Plasmid kodierte Proinsulin dient als Vorstufe für die Verbindung der Form His (A8), Gly (A21), Arg (B31), Arg (B32) - NH₂ - Humaninsulin. Die Prozessierung des Präproinsulins zu His (A8), Gly (A21), Arg (B31) als Zwischenstufe vor Amidierung erfolgt dabei wie in WO91/03550 beschrieben. Dabei setzt man herkömmliches Trypsin ein.

### Beispiel 21: Formulierung der amidierten Insulinderivate

Um die erfindungsgemäßen Insulinderivate auf ihre biologisch, pharmakologischen und physikalisch chemischen Eigenschaften zu testen, wurde von den Verbindungen wie folgt eine Lösung hergestellt: Das erfindungsgemäße Insulinderivat wurde mit einer Zielkonzentration von 240 ± 5 µM in 1 mM Salzsäure mit 80 µg/mL Zink (als Zinkchlorid) aufgelöst. Hierzu wurde von dem gefriergetrockneten Material zunächst eine um etwa 30% höhere Menge als aufgrund des Molekulargewichts und der angestrebten Konzentration benötigt eingewogen. Danach wurde die vorliegende Konzentration mittels analytischer HPLC bestimmt und die Lösung anschließend auf das zur Erreichung der Zielkonzentration erforderliche Volumen mit 5 mM Salzsäure mit 80 µg/mL Zink aufgefüllt. Falls erforderlich, wurde dabei der pH-Wert auf 3,5 ± 0,1 nachjustiert. Nach der endgültigen Analyse durch HPLC zur Absicherung der Zielkonzentration von 240 ± 5 µM wurde die fertige Lösung mittels einer Spritze mit einem 0,2 µm Filtervorsatz in ein mit einem Septum und einer Bördelkappe verschlossenes steriles Fläschchen überführt. Für die kurzfristige, einmalige Testung der erfindungsgemäßen Insulinderivate wurde keine Optimierung der Formulierungen, z.B. hinsichtlich eines Zusatzes isotonischer Agentien, Konservierungsmittel oder Puffersubstanzen, vorgenommen.

### Beispiel 22: Herstellung von His (A8), Gly (A21), Arg(B31) Humaninsulin mittels Hefeexpression

EP-A 1 364 032 beschreibt die Expression und Sekretion eines Fusionsporteins aus Hirudin und Miniproinsulin durch Hefe. Als besonders vorteilhafte Hefewirtsorganismen werden dabei S. cerevisiae, K. lactis, H. polymorpha und P. pastoris benannt. In Beispiel 4 der genannten Patentanmeldung ist die Konstruktion eines Plasmides beschrieben, das die Herstellung des Fusionsprotein in P. pastoris erlaubt und dessen DNA als Template zur Herstellung eines Miniproinsulins dient, dessen Aminosäuresequenz in der A- Kette in den Positionen A8, A15, A18 und A21 durch die Aminosäuren Histidin (A8), Glutaminsäure (A15), Asparaginsäure (A18) und Glycin (A21) charakterisiert ist. Verwendet wird der Primer pichia_H_if1. Neu synthetisiert und nach Synthese gereinigt werden folgende Primer:
pichia_G21_rev
   5'-TTTTTTGGCG CCGAATTCAC TATTA**GCC**AC *AGTAGTTTTC CAGCTGGTA*-3' (SEQ ID NO: 24)
   Das fett hervorgehobene Codon markiert die Positionen A 21.
pichia_H8_f mit der Sequenz
   5'-GAACAATGTTGT**CAT**AGTATCTGTTCTTTG*TACCAGCTGGAAAACTACTG*-3' (SEQ ID NO: 25)
   Das fett hervorgehobene Codon markiert die Positionen A 8.
pichia-a1-12rev
   5'- GAACAGATACT**ATG**ACAACATTGTTCAACGATACC -3' (SEQ ID NO: 26)
   Das fett hervorgehobene Codon markiert die Positionen A 8.

Äquimolare Mengen (1µg) an Primer pichia_G21_rev und Primer pichia_H8f, die sich partial überdecken, werden in Gegenwart einer thermophilen Polymerase und Polymerasepuffer, der die 4 Deoxynukleotide dATP, dCTP, dGTP und dTTP 5' enthält, bei 95°C denaturiert, anschließend 10 - 15 Minuten bei 56°C inkubiert. Das Reaktionsvolumen dieser Reaktion 1 beträgt 25µl. In einer Standard Polymerasekettenreaktion (Reaktion 2) werden parallel die Primer pichia_H_if1 und pichia-a1-12rev mit der beschriebenen Template DNA umgesetzt. In einer weiteren Polymerasekettenreaktion werden die Reaktionsprodukte der Reaktionen 1 und 2 als Template mit den Primern pichia_H_if1 / pichia_G21rev umgesetzt. Das Reaktionsprodukt dieser Reaktion wird mittels Gelelektrophorese gereinigt. Nach Umsetzung mit den Restriktionsenzymen Xhol und Sacll wird das DNA- Fragment, welches das Fusionsprodukt überdeckt in, Analogie zu Beispiel 4 in den beschrieben Vektor pPICZαA eingesetzt. Es entsteht das Plasmid pPIC_ins202. Unter Anwendung des beschriebenen kommerziell erhältlichen Expressions - Kits erfolgt die Expression des von dem Plasmid kodierten Fusionsproteins. Nach Herstellung der His (A8), Gly (A21), Arg (B31) - Humaninsulinvorstufe erfolgt nun die Kupplung von Arginin - Amid entsprechend der Beschreibung an Arginin (B31). Es entsteht die Verbindung His (A8), Gly (A21), Arg (B31), Arg (B32) - NH₂ - Insulin (YKL203), die ein neues Verzögerungsinsulin entsprechend Beispiel 20 darstellt und im Rattenversuch getestet wird. Die Kupplung mit Lysin - Amid führt in Analogie zu der Verbindung YKL203b, die als His (A8), Gly (A21), Arg (B31), Lys (B32) - NH₂-Insulin charakterisiert ist.

Die Verbindung kann aber auch als Zwischenstufe zur Darstellung der Verbindung Arg (A0), His (A8), Gly (A21), Arg (B31) Arg(B32) - NH₂ - Insulin, die in Anlehnung an Kohn et al. [Peptides 28 (2007) 935-948] hergestellt wird, dienen. Dazu werden 200mg der Verbindung His (A8), Gly (A21), Arg (B31), Arg (B32) - NH₂-Humaninsulin in 40 ml eines Gemisches von 100 mM Na₂HPO₄ / CH₃CN (50:50) gelöst und ein pH von 7,7 - 8,2 eingestellt.

Als weiteres Material wird ein Boc-Arg - NHS Ester hergestellt, indem man 0,3 mM Boc-Arg-OH, 0,4 mM N-hydroxysuccineimide (NHS) und 0,4 mM Dicyclohexylcarbodiimid (DCC) in Dichlormethan 40 Minuten mischt. Das Reaktionsgemisch wird im Rotationsvakuumverdampfer zur Trockene eingeengt. Das Gemisch wird anschließend in 5 ml Methanol aufgenommen und dem gelösten Ausgangsinsulin zugesetzt. Die Reaktion wird bei Raumtemperatur mindestens 60, aber maximal 120 Minuten gerührt. Die Reaktion wird durch Zugabe von 200µl TFA gestoppt. Die Masse der Reaktionsprodukte wird über LC-MS massenspektroskopisch charakterisiert, um den Nachweis zu erbringen, dass monoacyliertes Produkt, das aus drei Komponenten besteht, vorliegt. Die Komponenten werden über HPLC getrennt. Dabei werden auch di- acylierte und triacylierte Nebenprodukte abgetrennt. Die Fraktionen der HPLC - Trennung werden massenspektrokopisch analysiert. Die drei Fraktionen , die jeweils monoacyliertes Produkt enthalten, werden einer Aminosäuresequenzanalyse unterzogen, deren Interpretation die Identifikation der HPLC - Fraktion erlaubt, die die gewünschte Verbindung enthält von der anschließend durch schonende Hydrolyse die Boc-Schutzgruppen entfernt werden. Nach erneuter HPLC- Reinigung wird das gewünschte Produkt auf seine biologischen Eigenschaften getestet.

### Beispiel 23: Evaluierung der blutzuckersenkenden Wirkung von neuen Insulinanaloga in der Ratte

Die blutzuckersenkende Wirkung von ausgewählten neuen Insulinanaloga wird in männlichen, gesunden, normoglykämischen Wistarratten geprüft. Männlichen Ratten wird eine Dosis von 9 nmol/kg eines Insulinanalogons subkutan injiziert. Unmittelbar vor der Injektion des Insulinanalogons und in regelmäßigen Abständen bis zu acht Stunden nach der Injektion werden den Tieren Blutproben entnommen und darin der Blutzuckergehalt bestimmt. Das Experiment zeigt deutlich (vgl. Fig. 1), dass das erfindungsgemäße Insulinanalogon zu einem deutlich verzögerten Wirkeintritt und einer längeren, gleichmäßigen Wirkdauer führt.

### Beispiel 24: Evaluierung der blutzuckersenkenden Wirkung von neuen Insulinanaloga im Hund

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt. Das Experiment zeigt deutlich, dass das erfindungsgemäße Insulinanalogon zu einem deutlich verzögerten, flachen Wirkeintritt und einer längeren, gleichmäßigen Wirkdauer führt.

## Patentansprüche

1. Insulinanalogon ausgewählt aus der Gruppe enthaltend Arg (A0), His (A8), Gly (A21), Arg (B31), Arg (B32) - NH₂ - Humaninsulin, Arg (A0), Glu (A15), His (A8), Gly (A21), Arg (B31), Arg (B32) - NH₂ - Humaninsulin, His (A8), Gly (A21), Arg (B31), Arg (B32) - NH₂ - Humaninsulin.

2. Verfahren zur Herstellung eines Insulinanalogs gemäß Anspruch 1, wobei ein Vorläufer des Insulinanalogs rekombinant hergestellt wird, der Vorläufer enzmatisch zu zwei -kettigem Insulin prozessiert wird und eine Kupplung mit Argininamid in Gegenwart eines Enzyms mit Trypsinaktivität durchgeführt wird, und das Insulinanalogon isoliert wird.

3. Verwendung eines Insulinanalogs nach Anspruch 1 zur Herstellung eines Medikaments zur Behandlung von Diabetes Mellitus.

4. Verwendung gemäß Anspruch 3 in einem Verfahren zur Herstellung eines Medikaments zur Behandlung von Diabetes MellitusTyp I oder Typ II, zur Knorpelregeneration oder zur Unterstützung der beta -Zellregeneration.

5. Arzneimittel enthaltend ein Insulinanalog gemäß Anspruch 1 und/oder physiologisch verträgliche Salze davon.

6. Formulierung des Insulinanalogons nach Anspruch 1, wobei die Formulierung in wässriger Form enthaltend das gelöste Insulinanalogon vorliegt.

7. Formulierung des Insulinanalogons gemäß Anspruch 1, wobei die Formulierung in Form von Pulver vorliegt.

8. Formulierung gemäß Anspruch 7, wobei das Insulinanalogon gemäß Anspruch 1 in kristalliner und/oder amorpher Form vorhanden ist.

9. Formulierung des Insulinanalogons gemäß Anspruch 1, wobei die Formulierung in Form einer Suspension vorliegt.

10. Formulierung des Insulinanalogons gemäß Anspruch 1, wobei die Formulierung zusätzlich ein chemisches Chaperon enthält.

11. Formulierung nach einem oder mehreren der Ansprüche 6 bis 10, bei der noch zusätzlich ein Glucagon-Like Peptide-1 (GLP1) oder ein Analogon oder Derivat davon, oder Exendin-3 bzw. -4 oder ein Analogon oder Derivat davon enthalten ist.

12. Formulierung nach Anspruch 11, bei dem zusätzlich Exendin-4 enthalten ist.

13. Formulierung gemäß Anspruch 11, bei dem ein Analogon von Exendin-4 ausgewählt wird aus einer Gruppe enthaltend
H-desPro³⁶-Exendin-4-Lys₆-NH₂,
H-des(Pro^{36,37})-Exendin-4-Lys₄-NH₂ und
H-des(Pro^{36,37})-Exendin-4-Lys₅-NH₂,
oder ein pharmakologisch tolerierbares Salz davon.

14. Formulierung gemäß Anspruch 11, bei dem ein Analogon von Exendin-4 ausgewählt wird aus einer Gruppe enthaltend
desPro³⁶[Asp²⁸]Exendin-4 (1-39),
desPro³⁶ [IsoAsp²⁸]Exendin-4 (1-39),
desPro³⁶ [Met(O)¹⁴, Asp²⁸]Exendin-4 (1-39),
desPro³⁶ [Met(O)¹⁴, IsoAsp²⁸]Exendin-4 (1-39),
desPro³⁶ [Trp(O₂)²⁵, Asp²⁸]Exendin-2 (1-39),
desPro³⁶ [Typ(O₂)²⁵, IsoAsp²⁸]Exendin-2 (1-39),
desPro³⁶ [Met(O)¹⁴Trp(O₂)²⁵, Asp²⁸]Exendin-4 (1-39) und
desPro³⁶ [Met(O)¹⁴Trp(O₂)²⁵, IsoAsp²⁸]Exendin-4 (1-39),
oder ein pharmakologisch tolerierbares Salz davon.

15. Formulierung gemäß Anspruch 11, bei denen an die C-Termini der Analoga von Exendin-4 das Peptid -Lys₆-NH₂ angefügt ist.

16. Formulierung gemäß Anspruch 11, bei dem ein Analogon von Exendin-4 ausgewählt wird aus einer Gruppe enthaltend
H-(Lys)₆- des Pro³⁶ [Asp²⁸]Exendin-4(1-39)-Lys₆-NH₂
des Asp²⁸Pro³⁶, Pro³⁷, Pro₃₈ Exendin-4(1-39) -NH₂,
H-(Lys)₆- des Pro³⁶, Pro³⁷, Pro³⁸ [Asp²⁸]Exendin-4(1-39) -NH₂,
H-Asn-(Glu)₅ des Pro³⁶, Pro³⁷, Pro³⁸ [Asp²⁸]Exendin-4(1-39) -NH₂,
des Pro³⁶, Pro³⁷, Pro³⁸ [Asp²⁸]Exendin-4(1-39)-(Lys)₆-NH₂,
H-(Lys)₆- des Pro³⁶, Pro³⁷, Pro³⁸ [Asp²⁸]Exendin-4(1-39)-(Lys)₆-NH₂,
H-Asn-(Glu)₅- des Pro³⁶, Pro³⁷, Pro³⁸ [Asp²⁸]Exendin-4(1-39)-(Lys)₆-NH₂,
H-(Lys)₆- des Pro³⁶ [Trp(O₂)²⁵, Asp²⁸]Exendin-4(1-39)-Lys₆-NH₂,
H₋ des Asp²⁸ Pro³⁶, Pro³⁷, Pro³⁸ [Trp(O₂)²⁵]Exendin-4(1-39) -NH₂,
H-(Lys)₆- des Pro³⁶, Pro³⁷, Pro³⁸ [Trp(O₂)²⁵, Asp²⁸]Exendin-4(1-39) -NH₂,
H-Asn-(Glu)₅- des Pro³⁶, Pro³⁷, Pro³⁸ [Trp(O₂)²⁵, Asp²⁸]Exendin-4(1-39) -NH₂,
des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶, Pro³⁷, Pro³⁸ [Trp(O₂)²⁵, Asp²⁸]Exendin-4(1-39)-(Lys)₆-NH₂,
H-Asn-(Glu)₅- des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶ [Met(O)¹⁴, Asp²⁸]Exendin-4(1-39)-Lys₆-NH₂,
des Met(O)¹⁴ Asp²⁸ Pro³⁶, Pro³⁷, Pro³⁸ Exendin-4(1-39) -NH₂,
H-(Lys)₆- des Pro³⁶, Pro³⁷, Pro³⁸ [Met(O)¹⁴, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Asp²⁸]Exendin-4(1-39)-(Lys)₆-NH₂,
H-(Lys)₆- des Pro³⁶, Pro³⁷, Pro³⁸ [Met(O)¹⁴, Asp²⁸]Exendin-4(1-39)-Lys₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,
H-(Lys)₆- des Pro³⁶ [Met(O)¹⁴, Trp(O₂)²⁵, Asp²⁸]Exendin-4(1-39)-Lys₆-NH₂,
des Asp²⁸ Pro³⁶, Pro³⁷, Pro³⁸ [Met(O)¹⁴, Trp(O₂)²⁵]Exendin-4(1-39) -NH₂,
H-(Lys)₆- des Pro^{36,} Pro³⁷, Pro³⁸ [Met(O)¹⁴, Trp(O₂)²⁵, Asp²⁸]Exendin-4(1-39)-NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Trp(O₂)²⁵, Asp²⁸]Exendin-4(1-39)-(Lys)₆-NH₂,
H-(Lys)₆- des Pro^{36,} Pro³⁷, Pro³⁸ [Met(O)¹⁴, Trp(O₂)²⁵, Asp²⁸]Exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸] Exendin-4(1-39)-(Lys)₆-NH₂,
oder ein pharmakologisch tolerierbares Salz davon.

17. Formulierung nach Anspruch 11, bei dem zusätzlich Arg³⁴, Lys²⁶ (N^{ε}(γ-glutamyl(N^{α}-hexadecanoyl))) GLP-1 (7-37) [liraglutide] enthalten ist.

## Claims

1. Insulin analogue selected from the group containing
Arg (A0), His (A8), Gly (A21), Arg (B31), Arg (B32) - NH₂ - human insulin,
Arg (A0), Glu (A15), His (A8), Gly (A21), Arg (B31), Arg (B32) - NH₂ - human insulin,
His (A8), Gly (A21), Arg (B31), Arg (B32) - NH₂ - human insulin.

2. Process for preparing an insulin analogue according to claim 1, wherein a precursor of the insulin analogue is prepared recombinantly, the precursor is processed enzymatically to form two-chain insulin, and a coupling with argininamide is carried out in the presence of an enzyme having trypsin activity, and the insulin analogue is isolated.

3. Use of an insulin analogue according to claim 1 for preparing a medicine for treating diabetes mellitus.

4. Use according to claim 3 in a process for preparing a medicine for the treatment of type I or type II diabetes mellitus, for cartilage regeneration or for assisting beta cell regeneration.

5. Pharmaceutical preparation containing an insulin analogue according to claim 1 and/or a physiologically acceptable salt thereof.

6. Formulation of the insulin analogue according to claim 1, wherein the formulation is in aqueous form containing the dissolved insulin analogue.

7. Formulation of the insulin analogue according to claim 1, wherein the formulation is in the form of a powder.

8. Formulation according to claim 7, wherein the insulin analogue according to claim 1 is present in crystalline and/or amorphous form.

9. Formulation of the insulin analogue according to claim 1, wherein the formulation is in the form of a suspension.

10. Formulation of the insulin analogue according to claim 1, wherein the formulation additionally contains a chemical chaperone.

11. Formulation according to one or more of claims 6 to 10, which additionally contains a glucagon-like peptide-1 (GLP1) or an analogue or derivative thereof, or exendin-3 or -4 or an analogue or derivative thereof.

12. Formulation according to claim 11, which additionally contains exendin-4.

13. Formulation according to claim 11, wherein an analogue of exendin-4 is selected from a group containing
H-desPro³⁶-exendin-4-Lys₆-NH₂,
H-des(Pro^{36,37})-exendin-4-Lys₄-NH₂ and
H-des(Pro^{36,37})-exendin-4-Lys₅-NH₂,
or a pharmacologically tolerable salt thereof.

14. Formulation according to claim 11, wherein an analogue of exendin-4 is selected from a group containing
desPro³⁶[Asp²⁸]exendin-4 (1-39),
desPro³⁶[IsoAsp²⁸]exendin-4 (1-39),
desPro³⁶[Met(O)¹⁴, Asp²⁸]exendin-4 (1-39),
desPro³⁶[Met(O)¹⁴, IsoAsp²⁸]exendin-4 (1-39),
desPro³⁶[Trp(O₂)²⁵, Asp²⁸]exendin-2 (1-39),
desPro³⁶[Trp(O₂)²⁵, IsoAsp²⁸]exendin-2 (1-39),
desPro³⁶[Met(O)¹⁴Trp(O₂)²⁵, Asp²⁸]exendin-4 (1-39) and
desPro³⁶[Met(O)¹⁴Trp(O₂)²⁵, IsoAsp²⁸]exendin-4 (1-39),
or a pharmacologically tolerable salt thereof.

15. Formulation according to claim 11, wherein the peptide -Lys₆-NH₂ is attached to the C termini of the analogues of exendin-4.

16. Formulation according to claim 11, wherein an analogue of exendin-4 is selected from a group containing
H-(Lys)₆-des Pro³⁶ [Asp²⁸]exendin-4(1-39)-Lys₆-NH₂
des Asp²⁸Pro³⁶, Pro³⁷, Pro³⁸ exendin-4(1-39) -NH₂,
H-(Lys)₆-des Pro³⁶, Pro³⁷, Pro³⁸ [Asp²⁸]exendin-4(1-39) -NH₂,
H-Asn-(Glu)₅ des Pro³⁶, Pro³⁷, Pro³⁸ [Asp²⁸]exendin-4(1-39) -NH₂,
des Pro³⁶, Pro³⁷, Pro³⁸ [Asp²⁸]exendin-4(1-39)-(Lys)₆-NH₂,
H-(Lys)₆-des Pro³⁶, Pro³⁷, Pro³⁸ [Asp²⁸]exendin-4(1-39)-(Lys)₆-NH₂,
H-Asn-(Glu)₅-des Pro³⁶, Pro³⁷, Pro³⁸ [Asp²⁸]exendin-4(1-39)-(Lys)₆-NH₂,
H-(Lys)₆-des Pro³⁶ [Trp(O₂)²⁵, Asp²⁸]exendin-4(1-39)-Lys₆-NH₂,
H-des Asp²⁸ Pᵣo³⁶, Pro³⁷, Pro³⁸ [Trp(O₂)²⁵]exendin-4(1-39) -NH₂,
H-(Lys)₆-des Pro³⁶, Pro³⁷, Pro³⁸ [Trp(O₂)²⁵, Asp²⁸]exendin-4(1-39) -NH₂,
H-Asn-(Glu)₅-des Pro³⁶, Pro³⁷, Pro³⁸ [Trp(O₂)²⁵, Asp²⁸]exendin-4(1-39) -NH₂,
des Pro³⁶, Pro³⁷, Pro³⁸ [Trp(O₂)²⁵, Asp²⁸]exendin-4(1-39)-(Lys)₆-NH₂,
H-(Lys)₆-des Pro³⁶, Pro³⁷, Pro³⁸ [Trp(O₂)²⁵, Asp²⁸]exendin-4(1-39)-(Lys)₆-NH₂,
H-Asn-(Glu)₅-des Pro³⁶, Pro³⁷, Pro³⁸ [Trp(O₂)²⁵, Asp²⁸]exendin-4(1-39)-(Lys)₆-NH₂,
H-(Lys)₆-des Pro³⁶ [Met(O)¹⁴, Asp²⁸]exendin-4(1-39)-Lys₅-NH₂,
des Met(O)¹⁴ Asp²⁸ Pro³⁶, Pro³⁷, Pro³⁸ exendin-4(1-39) -NH₂,
H-(Lys)₆-des Pro³⁶, Pro³⁷, Pro³⁸ [Met(O)¹⁴, Asp²⁸]exendin-4(1-39) -NH₂,
H-Asn-(Glu)₅-des Pro³⁶, Pro³⁷, Pro³⁸ [Met(O)14, Asp²⁸] exendin-4(1-39) -NH₂,
des Pro³⁶, Pro³⁷, Pro³⁸ [Met(O)¹⁴, Asp²⁸]exendin-4(1-39)-(Lys)₆-NH₂,
H-(Lys)₆-des Pro³⁶, Pro³⁷, Pro³⁸ [Met(O)¹⁴, Asp²⁸]exendin-4(1-39)-Lys₆-NH₂,
H-Asn-(Glu)₅ des Pro³⁶, Pro³⁷, Pro³⁸ [Met(O)¹⁴, Asp²⁸] exendin-4(1-39)-(Lys)₆-NH₂,
H-(Lys)₆-des Pro³⁶[Met(O)¹⁴, Trp(O₂)²⁵, Asp²⁸]exendin-4(1-39)-Lys₆-NH₂,
des Asp²⁸ Pro³⁶, Pro³⁷, Pro³⁸ [Met(O)¹⁴, Trp(O₂)²⁵]exendin-4(1-39) -NH₂,
H-(Lys)₆-des Pro³⁶, Pro³⁷, Pro³⁸ [Met(O)¹⁴, Trp(O₂)²⁵, Asp²⁸]exendin-4(1-39) -NH₂,
H-Asn-(Glu)₅-des Pro³⁶, Pro³⁷, Pro³⁸ [Met(O)¹⁴, Asp²⁸] exendin-4(1-39) -NH₂,
des Pro³⁶, Pro³⁷, Pro³⁸ [Met(O)¹⁴, Trp(O₂)²⁵, Asp²⁸]exendin-4(1-39)-(Lys)₆-NH₂,
H-(Lys)₆-des Pro³⁶, Pro³⁷, Pro³⁸ [Met(O¹⁴, Trp(O₂)²⁵, Asp²⁸]exendin-4(1-39)-(Lys)₆-NH₂,
H-Asn-(Glu)₅-des Pro³⁶, Pro³⁷, Pro³⁸ [Met(O)¹⁴, Trp(O₂)²⁵, Asp²⁸] exendin-4(1-39)-(Lys)₆-NH₂,
or a pharmacologically tolerable salt thereof.

17. Formulation according to claim 11, which additionally contains Arg³⁴, Lys²⁶ (N^{ε}(γ-glutamyl(N^{α}-hexadecanoyl))) GLP-1 (7-37) [liraglutide].

## Revendications

1. Analogue de l'insuline choisi parmi le groupe comprenant
Arg (A0), His (A8), Gly (A21), Arg (B31), Arg (B32) - NH₂ - insuline humaine,
Arg (A0), Glu (A15), His (A8), Gly (A21), Arg (B31), Arg (B32) - NH₂-insuline humaine,
His (A8), Gly (A21), Arg (B31), Arg (B32) - NH₂- insuline humaine.

2. Procédé pour la préparation d'un analogue de l'insuline selon la revendication 1, dans lequel un précurseur de l'analogue de l'insuline est préparé par technologie recombinante, le précurseur est transformé enzymatiquement en une insuline à deux chaines et un couplage est réalisé avec l'arginine amide en présence d'une enzyme à activité trypsinique, et l'analogue de l'insuline est isolé.

3. Utilisation d'un analogue de l'insuline selon la revendication 1 pour la préparation d'un médicament pour le traitement du diabète sucré.

4. Utilisation selon la revendication 3 dans un procédé pour la préparation d'un médicament pour le traitement du diabète sucré de type I ou de type II, pour la régénération du cartilage ou la promotion de la régénération des cellules béta.

5. Médicament comprenant un analogue de l'insuline selon la revendication 1 et/ou un sel de celui-ci physiologiquement acceptable.

6. Formulation de l'analogue de l'insuline selon la revendication 1, dans laquelle la formulation se présente sous forme aqueuse contenant l'analogue de l'insuline.

7. Formulation de l'analogue de l'insuline selon la revendication 1, dans laquelle la formulation se présente sous forme de poudre.

8. Formulation selon la revendication 7, dans laquelle l'analogue de l'insuline selon la revendication 1 est présent sous forme cristalline et/ou sous forme amorphe.

9. Formulation de l'analogue de l'insuline selon la revendication 1, dans laquelle la formulation se présente sous forme d'une suspension.

10. Formulation de l'analogue de l'insuline selon la revendication 1, dans laquelle la formulation contient de plus un chaperon chimique.

11. Formulation selon l'une quelconque des revendications 6 à 10, dans laquelle est contenu encore en plus un glucagon like-peptide-1 (GLP1) ou un analogue ou dérivé de celui-ci, ou l'exendine-3 ou -4 respectivement ou un analogue ou un dérivé de celui-ci.

12. Formulation selon la revendication 11, dans laquelle est contenu en plus l'exendine-4.

13. Formulation selon la revendication 11, dans laquelle un analogue de l'exendine-4 est choisi parmi un groupe contenant
H-desPro³⁶-exendine-4-Lys₆-NH₂,
H-des(Pro^{36,37})-exendine-4-Lys₄-NH₂,
H-des(Pro^{36,37})-exendine-4-Lys₅-NH₂,
ou un sel de celui-ci pharmaceutiquement tolérable.

14. Formulation selon la revendication 11, dans laquelle un analogue de l'exendine-4 est choisi parmi un groupe contenant
desPro³⁶[Asp²⁸]-exendine-4 (1-39),
desPro³⁶[IsoAsp²⁸]-exendine-4 (1-39),
desPro³⁶[Met(O)¹⁴,Asp²⁸]-exendine-4 (1-39),
desPro³⁶[Met(O)¹⁴, IsoAsp²⁸]-exendine-4 (1-39),
desPro³⁶[Tryp(O₂)²⁵, Asp²⁸]-exendine-2 (1-39),
desPro³⁶[Tryp(O₂)²⁵, IsoAsp²⁸]-exendine-2 (1-39),
desPro³⁶[Met(O)¹⁴Trp(O₂)²⁵, Asp²⁸]-exendine-4 (1-39), et
desPro³⁶[Met(O)¹⁴Trp(O₂)²⁵, IsoAsp²⁸]-exendine-4 (1-39),
ou un sel de celui-ci pharmaceutiquement tolérable.

15. Formulation selon la revendication 11, dans laquelle le peptide -Lys₆-NH₂ est ajouté à la partie C-terminale des analogues de l'exendine-4.

16. Formulation selon la revendication 11, dans laquelle un analogue de l'exendine-4 est choisi parmi le groupe contenant
H-(Lys)₆- des Pro³⁶ [Asp²⁸]exendine-4(1-39)-Lys₆-NH₂
des Asp²⁸ Pro³⁶, Pro³⁷, Pro³⁸ exendine-4(1-39)-NH₂,
H-(Lys)₆- des Pro³⁶, Pro³⁷, Pro³⁸ [Asp²⁸]exendine-4(1-39)-NH₂,
H-Asn-(Glu)₅- des Pro³⁶, Pro³⁷, Pro³⁸ [Asp²⁸]exendine-4(1-39)-NH₂,
des Pro³⁶, Pro³⁷, Pro³⁸ [Asp²⁸]exendine-4(1-39)-Lys₆-NH₂,
H-(Lys)₆- des Pro³⁶, Pro³⁷, Pro³⁸ [Asp²⁸]exendine-4(1-39)-(Lys₆)-NH₂,
H-Asn-(Glu)₅- des Pro³⁶, Pro³⁷, Pro³⁸ [Asp²⁸]exendine-4(1-39)- (Lys₆)-NH₂,
H-(Lys)₆- des Pro³⁶ [Trp(O₂)²⁵, Asp²⁸]exendine-4(1-39)-Lys₆-NH₂,
H-des Asp²⁸ Pro³⁶, Pro³⁷, Pro³⁸ [Trp(O₂)²⁵]exendine-4(1-39)-NH₂,
H-(Lys)₆- des Pro³⁶, Pro³⁷, Pro³⁸ [Trp(O₂)²⁵, Asp²⁸]exendine-4(1-39)-NH₂,
H-Asn-(Glu)₅- des Pro³⁶, Pro³⁷, Pro³⁸ [Trp(O₂)²⁵, Asp²⁸]exendine-4(1-39)-NH₂,
des Pro³⁶, Pro³⁷, Pro³⁸ [Trp(O₂)²⁵, Asp²⁸]exendine-4(1-39)-Lys₆-NH₂,
H-(Lys)₆- des Pro³⁶, Pro³⁷, Pro³⁸ [Trp(O₂)²⁵, Asp²⁸]exendine-4(1-39)-Lys₆-NH₂,
H-Asn-(Glu)₅- des Pro³⁶, Pro³⁷, Pro³⁸ [Trp(O₂)²⁵, Asp²⁸]exendine-4(1-39)-Lys₆-NH₂,
H-(Lys)₆- des Pro³⁶ [Met(O)¹⁴, Asp²⁸]exendine-4(1-39)-Lys₆-NH₂,
des Met(O)¹⁴ Asp²⁸ Pro³⁶, Pro³⁷, Pro³⁸ exendine-4(1-39)-NH₂,
H-(Lys)₆- des Pro³⁶, Pro³⁷, Pro³⁸ [Met(O)¹⁴, Asp²⁸]exendine-4(1-39)-NH₂,
H-Asn-(Glu)₅- des Pro³⁶, Pro³⁷, Pro³⁸ [Met(O)¹⁴, Asp²⁸]exendine-4(1-39)-NH₂,
des Pro³⁶, Pro³⁷, Pro³⁸ [Met(O)¹⁴, Asp²⁸]exendine-4(1-39) )-Lys₆-NH₂,
H-(Lys)₆- des Pro³⁶, Pro³⁷, Pro³⁸ [Met(O)¹⁴, Asp²⁸]exendine-4(1-39)- Lys₆-NH₂,
H-Asn-(Glu)₅ des Pro³⁶, Pro³⁷, Pro³⁸ [Met(O)¹⁴, Asp²⁸]exendine-4(1-39)-(Lys₆)-NH₂,
H-(Lys)₆- des Pro³⁶ [Met(O)¹⁴, Trp(O₂)²⁵, Asp²⁸]exendine-4(1-39)-Lys₆-NH₂,
des Asp²⁸ Pro³⁶, Pro³⁷, Pro³⁸ [Met(O)¹⁴, Trp(O₂)²⁵]exendine-4(1-39)-NH₂, H-(Lys)₆- des Pro³⁶, Pro³⁷, Pro³⁸ [Met(O)¹⁴, Trp(O₂)²⁵, Asp²⁸]exendine-4(1-39)-NH₂,
H-Asn-(Glu)₅- des Pro³⁶, Pro³⁷, Pro³⁸ [Met(O)¹⁴, Asp²⁸]exendine-4(1-39)-NH₂,
des Pro³⁶, Pro³⁷, Pro³⁸ [Met(O)¹⁴, Trp(O₂)²⁵, Asp²⁸]exendine-4(1-39) )-Lys₆-NH₂,
H-(Lys)₆- des Pro³⁶, Pro³⁷, Pro³⁸ [Met(O)¹⁴, Trp(O₂)²⁵, Asp²⁸]exendine-4(1-39) )-(LYS₆)-NH₂,
H-Asn-(Glu)₅ - des Pro³⁶, Pro³⁷, Pro³⁸ [Met(O)¹⁴, Trp(O₂)²⁵, Asp²⁸]exendine-4(1-39)- (Lys₆)-NH₂,
ou un sel de celui-ci pharmaceutiquement tolérable.

17. Formulation selon la revendication 11, contenant en plus Arg³⁴, Lys²⁶(N^{ε}(γ-glutamyl(N^{α}-hexadecanoyl))) GLP-1 (7-37) [liraglutide].
